(19)

```
Europäisches
Patentamt

European
Patent Office

Office européen
des brevets
```

(11) **EP 4 101 398 B1**

(12) **EUROPÄISCHE PATENTSCHRIFT**

(45) Veröffentlichungstag und Bekanntmachung des Hinweises auf die Patenterteilung:
**05.03.2025   Patentblatt 2025/10**

(21) Anmeldenummer: **21178420.2**

(22) Anmeldetag: **09.06.2021**

(51) Internationale Patentklassifikation (IPC):
*A61B 17/22* (2006.01)   *A61L 24/00* (2006.01)
*A61L 24/04* (2006.01)   *A61L 24/10* (2006.01)
*A61P 13/04* (2006.01)

(52) Gemeinsame Patentklassifikation (CPC):
(C-Sets verfügbar)
**A61L 24/0031; A61L 24/0089; A61L 24/04;
A61L 24/10; A61P 13/04;** A61L 2300/442   (Forts.)

(54) **MEHR-KOMPONENTEN-ZUSAMMENSETZUNG ZUR ENTFERNUNG VON PARTIKELN**

MULTICOMPONENT COMPOSITION FOR REMOVING PARTICLES

COMPOSITION À COMPOSANTS MULTIPLES DESTINÉE À L'ÉLIMINATION DES PARTICULES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**14.12.2022   Patentblatt 2022/50**

(73) Patentinhaber: **Purenum GmbH**
**28359 Bremen (DE)**

(72) Erfinder:
• **Stößlein, Sebastian**
  **28209 Bremen (DE)**
• **Grunwald, Ingo**
  **28865 Lilienthal (DE)**

(74) Vertreter: **Eisenführ Speiser**
**Patentanwälte Rechtsanwälte PartGmbB**
**Postfach 31 02 60**
**80102 München (DE)**

(56) Entgegenhaltungen:
**WO-A1-2014/173467      WO-A1-2014/173468
WO-A1-2020/115508**

• **KOBASLIJA MURIS ET AL: "Removable Colored Coatings Based on Calcium Alginate Hydrogels", BIOMACROMOLECULES, vol. 7, no. 8, 19 July 2006 (2006-07-19), US, pages 2357 - 2361, XP055865842, ISSN: 1525-7797, DOI: 10.1021/bm060341q**

**(Forts. nächste Seite)**

(52) Gemeinsame Patentklassifikation (CPC): (Forts.)

C-Sets
**A61L 24/10, C08L 5/04**

**Beschreibung**

Die vorliegende Erfindung betrifft eine gelbildende Mehr-Komponenten-Zusammensetzung umfassend eine Komponente enthaltend mindestens ein vernetzbares Polymer und eine Komponente enthaltend mindestens ein Vernetzungsmittel, wobei beide Komponenten mindestens einen Farbstoff enthalten, und ein vernetztes Gel, sowie eine solche Zusammensetzung bzw. ein solches Gel zur Anwendung in einem Verfahren zum Entfernen von unerwünschten Partikeln aus einem Patienten.

Weitere Aspekte der vorliegenden Erfindung oder in deren Zusammenhang, sowie bevorzugte Ausführungsformen sind nachfolgend sowie in den beigefügten Patentansprüchen beschrieben.

Die Anhäufung von unerwünschten Ausfallprodukten oder Ablagerungen diverser Art sind ein häufig auftretendes Problem im menschlichen Körper. Bekannte Formen hiervon sind unter anderem Galleinsteine oder Nierensteine. Galleinsteine treten in etwa 10 bis 15 % der erwachsenen Bevölkerung auf, wobei die westlichen Industrieländer besonders betroffen sind. Etwa die Hälfte der deutschen Bevölkerung im Alter von über 60 Jahren hat Gallensteine. Während Gallensteine oftmals asymptomatisch bleiben, kommt es jedoch in einigen Fällen zu Koliken mit starken Schmerzen, erhöhten Leberwerten und allgemeinen Krankheitssymptomen bis hin zur Gelbsucht (Ikterus).

Aus epidemiologischer Sicht stellt das Nierensteinleiden eine der häufigsten Erkrankung der Menschheit dar, deren Inzidenz in Deutschland im Jahr 2000 1,45 % betrug, was wiederum ca. 1.200.000 Neuerkrankungen pro Jahr entspricht. Allein in Deutschland kann insgesamt von ca. 750.000 Behandlungsfällen pro Jahr ausgegangen werden. Die Zahl der Behandlungen zur Steinentfernung in Deutschland wird auf ca. 400.000 / Jahr, davon ca. die Hälfte für Behandlungen von Rezidivsteinen, geschätzt. Die genannten Zahlen lassen sich weltweit auf die millionenfache Durchführung solcher Behandlungen extrapolieren. Mit einer Summe von über 1,5 Milliarden Euro stellt die Nierensteinerkrankung also einen erheblichen Kostenfaktor im deutschen Gesundheitswesen dar. Nierensteine können ebenfalls für starke Schmerzen sorgen, eine Nierenentzündung auslösen, die Nieren schädigen oder gar zu einem (in der Regel einseitigen) akuten Nierenversagen führen.

Speichelsteine können sich in allen Drüsen des Mundbereichs bilden. Häufig werden diese von selbst aus den Drüsenausführungsgängen gespült. Jedoch können solche Steine auch in den Drüsenausführungsgängen stecken bleiben und beispielsweise zu einer akuten, schmerzhaften Speicheldrüsenentzündung führen.

Die Ursachen beider Problematiken sind vielseitig und reichen von ungesunder, fettreicher Ernährung bzw. Flüssigkeitsmangel oder Bewegungsmangel über Erkrankungen wie Diabetes mellitus oder Gicht bis hin zu genetischer Veranlagung.

Häufig werden Speichel- Gallen-, Bauchspeicheldrüsen- oder Nierensteine erst in einem bereits fortgeschrittenen Stadium entdeckt, in der Regel dann, wenn die ersten (schwerwiegenderen) Symptome auftreten. Die Steine können sich in den Organen selbst oder in ableitenden Gängen befinden. Meist sind die entstandenen Steine dann bereits zu groß, als dass diese durch minimalinvasive Verfahren am Stück aus dem Körper entfernt werden können. In diesem Fall werden die entstandenen Steine zertrümmert und die einzelnen Fragmente davon entfernt, z.B. mit Hilfe eines Endoskops. In einigen Fällen bilden sich auch mehrere Steine, welche ebenfalls vollständig entfernt werden müssen.

Eine Methode zur Behandlung von Nierensteinen durch gezieltes Auflösen der Ablagerungen unter Verwendung von quartären Ammoniumsalzen wird beispielsweise in US 5,244,913 beschrieben.

Nicht nur im Fall von Speichel-, Gallen- oder Nierensteinen müssen mehrere unerwünschte Partikel aus einem Patienten entfernt werden. So ist auch in anderen Fällen, beispielsweise bei Trümmerbrüchen, hier Splitter des körpereigenen Knochens, oder Schürfwunden, hier beispielsweise Fremdkörper wie Steine, Metall-, Kunststoff- oder Holzsplitter bzw. Fragmenten davon, eine Vielzahl an Partikeln möglichst vollständig aus dem Patienten zu entfernen.

All diese Fälle haben gemein, dass eine Vielzahl an Partikeln möglichst unkompliziert, gleichzeitig aber auch möglichst vollständig aus dem Patienten entfernt werden muss.

Verlassen beispielsweise die Speichel-, Gallen-, Bauchspeicheldrüsen- oder Nierensteine den Körper nicht auf natürlichem Wege oder bestehen medizinische Indikationen zur sofortigen Therapie, stellt die Endoskopie (minimalinvasive Spiegelungstechniken) neben der extrakorporalen Stoßwellenbehandlung (ESWL) den therapeutischen "Gold-Standard" dar. Angesichts der zunehmenden Evidenz für schlechtere Ergebnisse der ESWL, werden endoskopische Verfahren bevorzugt angewendet. Es ist davon auszugehen, dass aktuell 60-70 % aller Steinpatienten endoskopisch behandelt werden. Diese Tendenz ist steigend. Unter Zuhilfenahme endoskopischer Techniken werden Nierensteine vor Ort zerkleinert und entfernt. Ein bisher ungelöstes Problem stellen insbesondere kleine Restfragmente (<2 mm) dar, die während der Behandlung nicht effektiv entfernt werden können. Verbliebene Nierensteinfragmente fungieren als "Kristallisationskeime" aus denen sich zu 70 % neue Steine entwickeln. Dies wiederum führt zu erneuten medizinischen Problemen und Behandlungsbedarf. Solche Fragmente wurden früher als klinisch irrelevante Restfragmente (CIRF) bezeichnet, wobei sich aber in den letzten Jahren deutlich zeigte, dass diese sehr wohl klinisch relevant sind.

Bei der Lithotripsie werden Nierensteine durch extrakorporale Stoßwellen oder endoskopisch eingeführte Laser- oder Druckluftsonden zertrümmert. Dabei entstehen unterschiedlich große Bruchstücke, die entweder mit Hilfe von Fassinstrumenten entfernt oder ausgespült werden können. Ein bei der Lithotripsie auftretendes Problem ist, dass sich die Fragmente während des Zertrümmerns verteilen können oder in schwer zugängliche Regionen gelangen.

WO 2005/037062 betrifft eine Methode, mit der Nierensteine mit Hilfe eines Polymerpfropfens in einem bestimmten Bereich eingeschlossen (nicht umschlossen) werden, wodurch Gewebeschäden durch die entstehenden Fragmente während der Zertrümmerung weitgehend verhindert werden können. Gemäß WO 2005/037062 wird auf zumindest einer Seite eines Nierensteins eine gelbildende Flüssigkeit in das Lumen injiziert, beispielsweise ein thermosensitives Polymer, welches bei Körpertemperatur einen Gelpfropfen bildet. Das Polymer kommt dabei in der Regel nicht in Kontakt mit dem Nierenstein, dient aber dazu, die Effizienz der Lithotripsie zu erhöhen, indem es ein Verschieben des Nierensteins verhindert, und das umgebende Gewebe vor Schädigung durch die Fragmentierung schützt. Eine Anwendung dieses Systems wird explizit nur außerhalb der Niere empfohlen bzw. erlaubt.

Ein Ansatz um Objekte, wie beispielsweise Blutgerinnsel, unter Verwendung eines Klebstoffes aus dem Körper zu entfernen, wird in US 2008/0065012 angegeben. Der Klebstoff wird dabei auf einer Oberfläche verteilt und mit Hilfe eines Katheters in den Körper eingeführt. Wenn das Objekt an der Oberfläche angehaftet ist, wird der Katheter wieder herausgezogen und nimmt das Objekt mit.

Auf biologischen Makromolekülen basierende Klebstoffe und insbesondere gelbildende Polymer-Systeme finden in der Medizintechnik zunehmend Anwendungen. Dabei ist ihre hohe Biokompatibilität eines der wichtigsten Auswahlkriterien.

In US 6,663,594 B2 wird ein Verfahren zur Immobilisation eines Objekts, beispielweise eines Nierensteins, im Körper beschrieben, bei dem eine gelbildende Flüssigkeit in den Körper injiziert wird. In Kontakt mit dem Objekt wird ein Gel gebildet, welches das Objekt zumindest teilweise erfasst und immobilisiert. Die Immobilisierung dient dazu, das Objekt anschließend fragmentieren zu können ohne eine Verteilung der Bruchstücke zu riskieren bzw. um das Objekt bzw. die Bruchstücke mit einem endoskopischen Werkzeug aus dem Körper zu entfernen. Dabei verhindert das Gel, dass das Objekt bzw. ein Bruchstück verrutscht und von dem Werkzeug nicht erfasst werden kann. Nach Entfernen des Objekts bzw. der Bruchstücke wird das Gel aufgelöst oder mittels eines endoskopischen Werkzeugs extrahiert. Nachteil dieser Methode ist, dass beim Zertrümmern der Nierensteine das bereits abgebundene Gel zerstört werden kann und dadurch wieder Fragmente freigesetzt werden können, oder dass einzelne Fragmente aus dem Polymer austreten. Ein Greifen der Fragmente im Gel ist mit den herkömmlichen Greifwerkzeugen nicht möglich. Zudem ist die beschriebene Prozedur sehr aufwendig, da die Steine oder Stein-Fragmente einzeln gefasst und entfernt werden. Im Ergebnis bleiben mit relativ großer Wahrscheinlichkeit einzelne Steinfragmente zurück.

Ein Problem bei der Lithotripsie ist insbesondere das Auftreten kleiner bis mittelgroßer Steinfragmente (insbesondere < 2 mm), auch als "Gries" bezeichnet, da diese Bruchstücke weder effizient gegriffen noch gespült werden können. Restfragmente dieser Größe rutschen durch die Maschen der Greifinstrumente (Fasszängchen oder -körbchen) und machen die Extraktion von Gries sehr zeitaufwändig und bei größeren Steinmassen praktisch undurchführbar. Das Zurückbleiben solcher Nierensteinfragmente führt jedoch in einem sehr hohen Prozentsatz der Fälle zur Bildung neuer Nierensteine, da die Bruchstücke bzw. Fragmente als "Kristallisationskeime" fungieren.

WO 2014/173467 beschreibt ein gelbildendes System, umfassend eine Komponente enthaltend ein oder mehrere vernetzbare Polymere, eine Komponente enthaltend ein oder mehrere Vernetzungsmittel und gegebenenfalls eine Komponente enthaltend magnetisierbare Teilchen. Das gelbildende System kann zur Entfernung von Harnsteinen eingesetzt werden. Dabei wird / werden der / die Harnsteine zertrümmert. Anschließend werden die Komponente enthaltend ein oder mehrere Vernetzungsmittel und die Komponente enthaltend magnetisierbare Teilchen gemischt und per Katheter über ein Endoskopiegerät in den Bereich des Harntrakts, der die Fragmente des bzw. der zertrümmerten Harnsteine(s) enthält, injiziert. Daraufhin wird die Komponente enthaltend ein oder mehrere vernetzbare Polymere zugegeben, wodurch sich ein Gel bildet. Das gefestigte Gel umschließt dabei teilweise oder vollständig die Harnsteine bzw. die Fragmente davon und kann mittels eines Greifinstruments über das Operationsendoskop mitsamt den Harnsteinen bzw. den Fragmenten davon entfernt werden.

Bei der Durchführung hat sich jedoch gezeigt, dass ein farbloses Gel einige Schwierigkeiten bei der Entfernung der Nierensteine bzw. deren Fragmente mit sich bringt. Die genaue Menge und der genaue Ort der injizierten Komponente(n) lässt sich nicht präzise kontrollieren. Auch kann nicht ermittelt werden, ob die Gelbildung bereits abgeschlossen ist und das gebildete farblose Gel lässt sich endoskopisch nur schwer wiederfinden. Zudem kann nicht festgestellt werden, ob die jeweiligen Komponenten durch die Verwendung einer Spüllösung oder einer zu starken Dosierung verwirbelt werden, wodurch die Komponenten gegebenenfalls so stark verdünnt werden, dass eine ordnungsgemäße Funktion nicht gewährleistet werden kann.

Um diese Probleme anzugehen, beschreibt Cloutier J, Cordeiro ER, Kamphuis GM, Villa L, Letendre J, de la Rosette JJ, Traxer O (2014) "The glueclot technique: a new technique description for small calyceal stone fragments removal" Urolithiasis 42 (5):441-444, autologes Blut zu verwenden. Allerdings muss das Blut zunächst entnommen werden, was einen weiteren Schritt bei der Anwendung erforderlich macht. Zudem dauert die Bildung eines Blutklumpens aus geronnenem Blut deutlich länger als die Bildung eines gefestigten Gels aus den Komponenten der WO 2014/173467. Auch erschwert der geringe Farbkontrast zwischen Blut und dem Gewebe des Patienten die Anwendung. Weiterhin wünscht der behandelnde Arzt die eingebetteten Steinfragmente zu sehen, als Erfolgskontrolle des Eingriffes. Im Blutklumpen ist dieses jedoch nicht machbar wegen der starken Eigenfarbe des Blutes. Eine Verdünnung des Blutes würde aber die Bildung eines festen Blutklumpens verhindern bzw. dessen Festigkeit stark reduzieren. Zusätzlich kann das Eigenblut nicht mit Hilfe eines feinen Katheters gezielt im Bereich der Steinfragmente z.B. in einem distalen Nierenkelch appliziert werden (Blut hat eine zu hohe Viskosität hierfür).

Das gelbildende System der WO 2014/173467 kann zusätzlich einen oder mehrere Farbstoffe enthalten, die es erleichtern, das gefestigte Gel bzw. eine, beide, mehrere oder sämtliche der verwendeten Komponenten (vor dem Abbinden) endoskopisch zu visualisieren.

Gleichermaßen beschreibt WO 2014/173468 ein Kit zum Herstellen eines vernetzten Gels, das Eisenoxidpartikel enthalten kann.

Es hat sich jedoch herausgestellt, dass der Einsatz vieler solcher Farbstoffe auch erhebliche Nachteile mit sich bringt, beispielsweise wenn das Gel vergleichbar mit einem Blutklumpen, wie beschrieben in Cloutier J, Cordeiro ER, Kamphuis GM, Villa L, Letendre J, de la Rosette JJ, Traxer O (2014) "The glueclot technique: a new technique description for small calyceal stone fragments removal" Urolithiasis 42 (5):441-444), nicht mehr transparent ist. Ein farbloses bzw. transparentes Gel bietet die Möglichkeit, das Umschließen der Nierensteine bzw. den Fragmenten davon während der Gelbildung zu kontrollieren. Sind die Nierensteine bzw. deren Fragmente nicht oder kaum umschlossen, führt die Entfernung des Gels nicht zur (vollständigen) Entfernung der Nierensteine bzw. derer Fragmente. Während des Eingriffs kann somit keine Erfolgskontrolle bei einem gefärbten Gel durchgeführt werden, was unter Umständen eine erneute Injektion der Komponenten und Gelbildung erfordert und den Eingriff signifikant verlängert.

Als besonders problematisch hat sich der Einsatz von Farbstoffen auch dadurch erwiesen, dass die eingesetzten Farbstoffe nicht nur das Gel, sondern auch das umgebende Gewebe anfärben. Das Gel kann durch einen Farbstoff endoskopisch zwar visualisiert werden, die vollständige Entfernung des Gels ohne aber eine Verletzung des umgebenden Gewebes wird dadurch aber drastisch erschwert.

Auch wurde gefunden, dass die Konsistenz des sich bildenden Gels durch viele Farbstoffe negativ beeinflusst wird. Hieraus ergibt sich die Gefahr, dass das entstandene Gel bei der Entfernung nicht fest genug ist und nur unter großem Aufwand mitsamt den Nierensteinen bzw. deren Fragmente endoskopisch entfernt werden kann. Im Gegensatz hierzu können bestimmte farbgebende Substanzen dazu führen, dass das Gel zu fest bzw. eine gummiartige Konsistenz bekommt. Dieses führt dazu, dass das gebildete Gel sich nicht oder nur schlecht auflöst bzw. nicht mehr mit Hilfe der Greifer in kleinere Stücke zerteilt werden kann.

Der Einsatz von Farbstoffen bringt somit zwar Vorteile mit sich, die bislang aber nur verwirklicht werden können, indem die beschriebenen Nachteile in Kauf genommen werden.

Entsprechende Probleme, sowie die Vor- und Nachteile bei dem Einsatz von Farbstoff(en) treten auch bei der Entfernung von Speichel-, Bauchspeicheldrüsen- oder Gallensteinen, sowie auch bei anderen Körpersteinen wie hierin beschrieben, auf und lassen sich ohne Weiteres auch auf andere Fälle, in denen Partikel aus einem Patienten zu entfernen sind, beispielsweise auf die oben beschriebenen weiteren Fälle, übertragen.

Die primäre Aufgabe der vorliegenden Erfindung war es daher, eine Möglichkeit bereitzustellen, bei welcher Partikel aus einem Patienten entfernt werden können, und bei welcher die oben genannten Vorteile, bzw. möglichst viele dieser Vorteile, ohne die aufgeführten Nachteile, bzw. mit möglichst wenigen der Nachteile, erzielt werden können.

Insbesondere war es Aufgabe der vorliegenden Erfindung, ein verbessertes Mittel bereitzustellen, dass dazu geeignet ist, Partikel zuverlässig aus dem Körper extrahieren zu können.

Die primäre Aufgabe der vorliegenden Erfindung wird gelöst durch eine gelbildende Mehr-Komponenten-Zusammensetzung, vorzugsweise Zwei-Komponenten-Zusammensetzung, zur Bildung eines Gels,

die Zusammensetzung bestehend aus oder umfassend

eine Komponente (A), enthaltend ein oder mehrere vernetzbare, vorzugsweise kationisch vernetzbare Polymere, und

eine Komponente (B), enthaltend ein oder mehrere Vernetzungsmittel zum Vernetzen des bzw. der vernetzbaren Polymere

wobei Komponente (A) und Komponente (B) mindestens einen Farbstoff umfassen, wobei der/die Farbstoff(e) in Komponente (A) unterschiedlich ist/sind von dem/den Farbstoff(en) in Komponente (B),

wobei der mindestens eine Farbstoff eine transparente Färbung des Gels und optional der Komponente (A) und/oder der Komponente (B) vornimmt,

wobei der mindestens eine Farbstoff das Vernetzen des bzw. der vernetzbaren Polymere bei in-Kontakt-Bringen der Komponenten (A) und (B) nicht beeinträchtigt,

wobei der mindestens eine Farbstoff aus dem gebildeten Gel nicht austritt,

und wobei der mindestens eine Farbstoff ein angefärbtes Molekül ausgewählt aus der Gruppe bestehend aus Dextranen, Polyethylenglycol, Stärke, Gellane, Polyelektrolyte wie Poly-l-lysin und andere Polyaminosäuren, wie z. B. Poly(ornithin), Poly(arginin), Poly(histidin); Polypeptide und Proteine wie z.B. Gelatine, sowie Polysaccharide wie Chitosan, Alginate wie Natriumalginat, Polyglykolsäure (PGA), Polymilchsäure (PLA), Poly-2-hydroxy-butyrat (PHB), Polycaprolacton (PCL), Poly(milch-co-glykol) säure (PLGA), Protaminsulfat, Spermidin, Albumin, Carrageenan, Furcellaran, Pektin, Xanthan, Hyaluronsäure, Natriumcarboxymethylcellulose, Heparin, Heparansulfat, Cellulosederivate oder Natriumcarboxymethylcellulose, Chondroitinsulfat, Dermatansulfat und Dextransulfat beinhaltet oder daraus besteht,

zur Anwendung in einem Verfahren zum Entfernen von unerwünschten Partikeln aus einem Patienten.

Der Begriff "gelbildende Zusammensetzung", wie hierin verwendet, ist vorzugsweise so zu verstehen, dass die Zusammensetzung nicht nur geeignet ist, ein Gel zu bilden, sondern dass durch Mischung der Komponenten der Zusammensetzung auch tatsächlich ein Gel entsteht.

Das durch Mischen der Komponenten der Zusammensetzung entstehende Gel ist vorzugsweise ein Hydrogel.

Zudem ist es bevorzugt, dass der bzw. mindestens einer des mindestens einen Farbstoffes einen hohen Kontrast zum Gewebe des Patienten, in welchem die Mehr-Komponenten-Zusammensetzung eingesetzt wird, hat. So ist es bevorzugt, dass der bzw. mindestens einer des mindestens einen Farbstoffes nicht für eine rote oder gelb-orangene Färbung sorgt.

Weiter ist es besonders bevorzugt, dass der Farbstoff physiologisch unbedenklich ist und somit in der Anwendung an / in einem Patienten unproblematisch eingesetzt werden kann.

Es wurde überraschend gefunden, dass einige Farbstoffe eine transparente Färbung ermöglichen, ohne die oben genannten negativen Merkmale aufzuweisen. Dabei kann einerseits das gefestigte Gel durch den Farbstoff angefärbt werden, andererseits aber auch die noch nicht gefestigte Mischung der Komponenten (A) und (B) sowie gegebenenfalls weiteren Komponenten.

Vorzugsweise färbt der Farbstoff das gefestigte Gel und/oder die Mischung der Komponenten (A) und (B) sowie gegebenenfalls weiteren Komponenten und/oder die Komponente(n) (A) und/oder (B) und/oder gegebenenfalls vorhandene weitere Komponenten mit einer transparenten Färbung. Vorzugsweise beinhaltet das gefestigte Gel den / die Farbstoff(e). Vorzugsweise beinhalten drei oder alle Komponenten mindestens einen Farbstoff. Vorzugsweise kann der bzw. können die Farbstoff(e) der jeweiligen Komponente (wie vorstehend beschrieben) auch kurz vor der Verwendung hinzugegeben werden.

Der Begriff "transparent" wie hierin verwendet beschreibt die Eigenschaft eines Gegenstands oder Materials (hier eines gefestigten Gels und ggf. der Komponente(n) (A), (B) und/oder gegebenenfalls weiterer Komponenten), lichtdurchlässig zu sein. Der Begriff, wie hierin verwendet, umfasst neben der vollständigen Lichtdurchlässigkeit auch die Eigenschaft, nur teilweise lichtdurchlässig (transluzent) zu sein. Das Gegenstück zu "transparent" ist "opak", d.h. lichtundurchlässig. Vorzugsweise beschreibt der Begriff "transparent" alle Materialien, die nicht "opak" sind. Die Berechnung der Transmission (Durchlässigkeit von Licht) sowie deren Kehrwert, der Opazität O, sowie die für die Messung der Lichtströme angewendeten Methoden sind dem Fachmann bekannt. Vorzugsweise wird die Transmission bzw. die Lichtströme gemessen durch die UV-Vis Spektroskopie. Die Transmission T berechnet sich allgemein als Verhältnis von einem auf ein Material einfallenden Lichtstrom ($I_0$) zu dem von diesem Material transmittierten Lichtstrom I:

$$T = I / I_0$$

$$O = I_0 / I$$

Ein Wert von T = 100 % bzw. O = 1 bedeutet vollständige Transparenz, wohingegen ein Wert von T = 0% bzw. O = ∞ vollständige Opazität beschreibt. Vorzugsweise beschreibt der Begriff "transparent" eine Transmission T von mehr als 15 %, bevorzugt von mindestens 16 %, besonders bevorzugt von mindestens 17 %, ganz besonders bevorzugt von mindestens 18 %.

Besonders bevorzugt wird die Transmission T mit einem UV-Vis Spektrometer gemessen, vorzugsweise durch Messung im sichtbaren Spektrum, besonders bevorzugt in einem Bereich von 400 bis 800 nm.

Vorzugsweise wird die Transmission T mit einem Verfahren gemessen, das die folgenden Schritte umfasst:

(i) Bereitstellen eines erfindungsgemäßen Gels,

(ii) Bereitstellen einer Referenz, vorzugsweise Wasser,

(iii) Messen der Transmission T mit einem Spektrometer, vorzugsweise mit einem UV-vis Spektrometer, vorzugsweise durch Messung im sichtbaren Spektrum, besonders bevorzugt in einem Bereich von 400 bis 800 nm.

Bevorzugt werden in dem Verfahren das Gel aus Schritt (i) und die Referenz aus Schritt (ii) in für die Messung geeignete Behälter, vorzugsweise Küvetten, eingebracht.

Weitere bevorzugte Merkmale werden in Beispiel 5 beschrieben, wobei ein Verfahren zur Messung der Transmission T eines, mehrere oder alle der Merkmale aus Beispiel 5 beinhalten oder daraus bestehen kann.

Durch eine transparente Färbung, wie hierin definiert, wird es dem Verwender ermöglicht, die Vorteile eines Farbstoffes zu nutzen. So ist die transparente Färbung hilfreich bei der Befüllung des Applikationsgerätes bzw. -hilfsmittels (z.B. einem Katheter). Ebenfalls hilft die transparente Färbung bei der Dosierung im Einsatzgebiet im Körper des Patienten: Die transparente Färbung zeigt an, wo die gefärbte Komponente gerade appliziert wird, wie viel appliziert wird und wo die gefärbte Komponente bereits appliziert wurde. Auch bei der Gelbildung ist eine transparente Färbung hilfreich, da so ersichtlich ist, ob es bei der Applikation zu Turbulenzen kommt. Nach Entfernen des gefestigten Gels samt der davon umschlossenen Partikel aus dem Bereich des Körpers des Patienten kann durch die transparente Färbung auch festgestellt werden, ob sich noch Gelbestandteile im Körper des Patienten befinden. Gleichzeitig kann durch eine transparente Färbung auch eine Erfolgskontrolle durchgeführt werden: Durch die transparente Färbung ist ersichtlich, ob die zu entfernenden Partikel bereits durch das Gel umschlossen sind bzw. welche der Partikel bereits umschlossen sind und wo noch ein solches Gel zur Entfernung der Partikel benötigt wird.

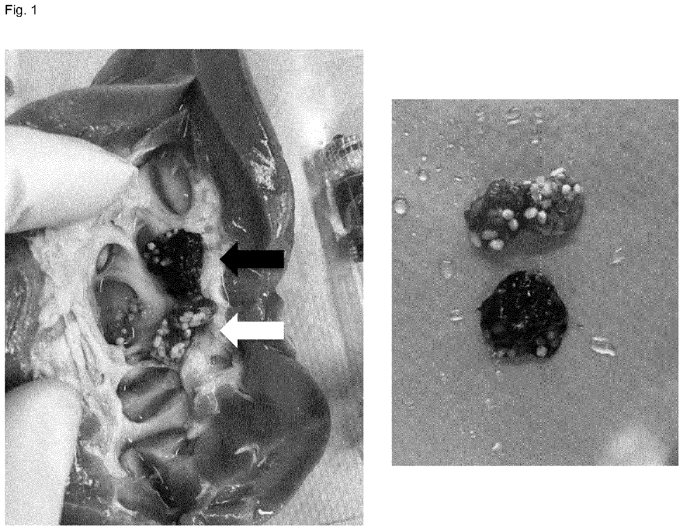

Es hat sich gezeigt, dass Farbstoffe, die eine weiße oder eine schwarze Färbung ermöglichen, eine Transparenz häufig nicht zulassen (vgl. Fig. 1). Daher ist es bevorzugt, dass der bzw. mindestens einer des mindestens einen Farbstoffes nicht für eine schwarze oder weiße Färbung sorgt.

Neben der Konzentration eines eingesetzten Farbstoffs hat überwiegend die Art des Farbstoffes einen Einfluss auf das Entstehen einer transparenten Färbung. So bestimmt vorwiegend die chemische Struktur wie u.a. die Elektronenverteilungen, die Ladungen oder deren molekulare Masse bzw. Zusammensetzung eines Farbstoffs dessen Eignung, eine transparente Färbung hervorzurufen.

Unter dem Begriff "das Vernetzen des bzw. der vernetzbaren Polymere bei in-Kontakt-Bringen der Komponenten nicht beeinträchtigen", wie hierin verwendet, wird verstanden, dass eine Vernetzung des bzw. der vernetzbaren Polymere bei in-Kontakt-Bringen der Komponenten weiterhin möglich ist und ein gefestigtes Gel entstehen kann. Durch den Zusatz von Substanzen wie beispielsweise Farbstoffen kann die Vernetzung langsamer erfolgen, gilt in diesem Fall aber nicht als beeinträchtigt, da eine Vernetzung weiterhin stattfindet. Vorzugsweise wird durch den bzw. die eingesetzten Farbstoff(e) die für die Vernetzung benötigte Zeit nicht oder im Wesentlichen nicht verändert. Vergleichbares gilt für die Möglichkeit, dass Gel wieder aufzulösen.

Besonders vorteilhaft ist es auch, wenn der Farbstoff nicht aus dem gefestigten Gel und/oder aus der bzw. einer Komponente enthaltend den Farbstoff austritt. In diesem Fall wird kein umliegendes Gewebe des Patienten angefärbt und der Farbstoff kann als Indikator für das Gel bzw. die bzw. eine Komponente enthaltend den Farbstoff dienen. Eine Erfolgskontrolle, ob das gefestigte Gel wieder vollständig aus dem Patienten entfernt wurde, ist daher möglich. Auch wird die Verwechslung von Gel und Gewebe des Patienten minimiert und bei der anschließenden Entfernung des Gels nur das Gel selbst samt der davon umschlossenen Partikel und nicht (körpereigenes) Gewebe des Patienten, das nicht entfernt werden soll, entfernt. Versehentliche Verletzungen des umliegenden Patientengewebes werden somit minimiert.

Vorzugsweise ist ein Farbstoff im Sinne der vorliegenden Erfindung eine chemische Struktur, welche Licht im Spektrum des sichtbaren Lichtes teilweise absorbiert und reflektiert und wiederum Licht einer spezifischen Wellenlänge bzw. eines spezifischen Wellenlängen-Musters im Bereich des sichtbaren Wellenbereiches remittiert. Ein Farbstoff enthält dabei einen Chromophor.

Besonders vorteilhaft haben sich Farbstoffe erwiesen, die selbst angefärbte große Moleküle beinhalten oder daraus bestehen. Solche Moleküle können ausgewählt sein aus der Gruppe bestehend aus Dextranen, vorzugsweise mit einer Molekülmasse von mindestens 10 kDa, besonders bevorzugt mindestens 20 kDa; Polyethylenglycol, Stärke, Gellane, Po-Iyelektrolyte wie Poly-I-Iysin und andere Polyaminosäuren, wie z. B. Poly(ornithin), Poly(arginin), Poly(histidin); Polypeptide und Proteine wie z.B. Gelatine, sowie Polysaccharide wie Chitosan, Alginate wie Natriumalginat, Polyglykolsäure (PGA), Polymilchsäure (PLA), Poly-2-hydroxy-butyrat (PHB), Polycaprolacton (PCL), Poly(milch-co-glykol) säure (PLGA), Protaminsulfat, Spermidin, Albumin, Carrageenan, Furcellaran, Pektin, Xanthan, Hyaluronsäure, Natriumcarboxymethylcellulose, Heparin, Heparansulfat, verschiedene Cellulosederivate oder Natriumcarboxymethylcellulose, Chondroitinsulfat, Dermatansulfat und Dextransulfat.

Solche Moleküle werden dann bevorzugt farblich markiert, sodass sie als erfindungsgemäße Farbstoffe dienen können. Solch eine Markierung kann insbesondere durch Reaktivfarbstoffe vorgenommen werden.

Reaktivfarbstoffe sind vorzugsweise zu verstehen als chemische Verbindungen mit einem Chromophor, welche eine oder mehrere Funktionen haben, die es ermöglichen eine kovalente Bindung zu knüpfen. Die Bindung erfolgt hierbei über einen "Reaktivanker" welcher beispielsweise aus halogensubstituierten aromatischen Heterocyclen, wie "Monochlortriazine", "Monofluortriazine", "Difluorpyrimidine", "Difluorchlorpyrimidine" und "Dichlorchinoxalincarbonamide", besteht oder solche umfasst.

Als Reaktivfarbstoffe gelten vorzugsweise auch Vinylsulfonfarbstoffe, welche über eine Vinylsulfon-Gruppe eine kovalente Bindung aufbauen, wie z.B. "C. I. Reactive Black 5, C. I. Reactive Blue 19, C. I: Reactive Orange 107, C. I. Reactive Red 239.

Für eine solche farbliche Markierung der Moleküle, wie oben beschrieben, eignen sich insbesondere diejenigen, die unter den Marken Cibacron (Ciba Ltd.) und Procion (ICI) verkauft werden, z.B. Cibacronblau (F3G-A), C.I. Reactive Blue 235, C.I. Reactive Blue 204, C.I. Reactive Blue 7, C.I. Reactive Red 1, Cibacron Orange G-E, Zibakronbrillantblau FBR-P, Zibakronblau F3G-A, Zibakronbrillantrot 3B-A, Zibakronbraun 3GR-A, Zibakronscharlachrot 2G, Zibakronscharlachrot 4G-P, Zibakronbrillantrot B-A, Procionbrillant Orange HGRS, Procion-Blau HBS, Procion-Brillantrot H7BS, Procion-Orange-Braun HGS, Procion-Scharlachrot H3GS, Procion-Rot H3B, Procion-Rot HE2B, Procion-Rot P3BN, Procion-Rot MX2B, Procion-Blau MX3G, Procion-Gelb MXR, Procion-Gelb H5G, Procion-Rot H8BN, Procion-Grün H-4G, Procion-Braun H-GR, Procion-Blau MX-G, Procion-Blau HE-RD, Procion-Blau H-B, Procion-Blau MXR, Procion-Gelb HA, Procion-Grün HE-4BD, Procion-Rot HE7B, Procion-Rot MX5B, Procion-Rot MY. 8 B, Procion Rubin MXB, Procion Scharlachrot MXG, Procion Orange MXG, Procion Gelb MX6G, Procion Braun H2G, Procion Gelb MX8G, Procion Türkis HA, Procion Türkis H7G, Procion Braun MX5BR, Procion Blau MX7RX, Procion Blau MX4G.

Vorzugsweise erfolgt eine solche farbliche Markierung durch kovalente Bindung einer solchen Substanz an die großen Moleküle wie vorangehend beschrieben. Als besonders bevorzugte erfindungsgemäße Farbstoffe hat sich insbesondere Dextranblau erwiesen. Vorzugsweise sind die Farbstoffe gebildet aus einer Kombination eines großen Moleküls, wie hierin beschrieben, und mindestens einer Markierung, wie hierin beschrieben.

Große Moleküle sind insbesondere vorteilhaft, da sich diese aufgrund ihrer Größe nicht aus dem gefestigten Gel lösen können. Bevorzugt ist der Farbstoff so groß, dass eine Bewegung des Farbstoffes durch das gefestigte Gel aufgrund der Größe des Farbstoffes im Wesentlichen verhindert wird. Vorzugsweise ist eine Bewegung des Farbstoffes durch das nicht gefestigte Gel möglich.

Vorzugsweise kann ein Farbstoff, wie hierin beschrieben, gemäß der vorliegenden Erfindung kovalent, ionisch, über hydrophobe Wechselwirkungen, über Wasserstoffbrückenbindungen und/oder über Van-der-Waals-Wechselwirkungen an das, ein, bzw. mehrere Polymer(e) der Komponente (A) und/oder der Komponente (B) gebunden sein, bzw. mit diesem interagieren.

Vorzugsweise kann ein Farbstoff, wie hierin beschrieben, gemäß der vorliegenden Erfindung kovalent oder ionisch an einen oder mehrere weiteren Bestandteil / weitere Bestandteile der Komponente (A) und/oder der Komponente (B) gebunden sein.

Besonders (aber nicht nur) für die Anfärbung der Komponente (B) eignen sich nicht nur Farbstoffe basierend auf großen Molekülen, wie hierin beschrieben, sondern auch kleinere Farbstoffe, sofern sich diese über physikalische Wechselwirkungen wie z.B. der Ladung in dem gefestigten Gel festhaften, wodurch vorzugsweise ebenfalls eine Bewegung des Farbstoffes durch das gefestigte Gel im Wesentlichen verhindert wird.

Vorteilhafterweise können die erfindungsgemäß eingesetzten Farbstoffe die Komponente(n) (A) und (B) anfärben, um deren Lokalisation und Dosierung zu erleichtern bzw. zu steuern. Zudem wird eine Verwechslung zwischen Zusammensetzung (A) und/oder (B) und/oder dem hergestellten Gel und dem Gewebe des Patienten vorteilhafterweise vermieden.

Komponente (A) und Komponente (B) umfassen mindestens einen Farbstoff und der/die Farbstoff(e) in Komponente (A) ist/sind von dem/den Farbstoff(en) in Komponente (B) unterschiedlich. Dies hat sich insbesondere dadurch als vorteilhaft erwiesen, da so farblich zwischen den Komponenten (A) und (B) unterschieden werden kann. Auch ist so vorzugsweise und vorteilhafterweise möglich, festzustellen, ob sich die Komponenten (A) und (B) bereits gemischt haben bzw. vermischt wurden, da durch eine Mischung der beiden Komponenten vorzugsweise eine Farbe entsteht, die sich von der Farbe der gefärbten Komponenten (A) und/oder (B) unterscheiden.

Vorzugsweise umfassen Komponente (A) und Komponente (B) jeweils mindestens einen Farbstoff umfassend ein großes Molekül, wie hierin beschrieben. Vorzugsweise umfasst nur Komponente (A) oder nur Komponente (B) mindestens einen Farbstoff umfassend ein großes Molekül, wie hierin beschrieben. Vorzugsweise umfasst nur Komponente (A) oder nur Komponente (B) mindestens einen Farbstoff umfassend ein großes Molekül, wie hierin beschrieben und die jeweils andere Komponente, Komponente (B) oder Komponente (A), einen kleineren Farbstoff, wie hierin beschrieben.

Die Polymere bzw. Polymer-Einheiten der Komponente (A) werden bevorzugt über ionische Wechselwirkungen (siehe Komponente (B)) vernetzt. Daher ist eine Vielzahl von Makromolekülen, die als Liganden ein- oder mehrwertiger Kationen auftreten und Chelatkomplexe bilden können, für die erfindungsgemäße Anwendung geeignet. Dazu gehören insbesondere Hydrogele, biokompatible zuckerbasierte (z.B. modifizierte Cellulosen) oder proteinogene Klebstoffe oder fibrin- oder kollagen basierte Systeme (besonders bevorzugte Polymere sind weiter unten beschrieben).

Vorzugsweise ist bzw. sind das bzw. ein, mehrere oder sämtliche vernetzbare Polymere der Komponente (A) ausgewählt aus der Gruppe bestehend aus Polysacchariden, insbesondere Polysacchariden mit deprotonierten oder deprotonierbaren funktionellen Gruppen, vorzugsweise Carboxy-Gruppen, bevorzugt Polysacchariden aus der Gruppe der Polyuronide, besonders bevorzugt Polysacchariden aus der Gruppe der Alginate und Pektine, und jeweils deren chemischen Derivaten.

Polysaccharide wie Dextrane, Carrageenane, Alginate, Gellane und Pektine sind für den Einsatz im Körper besonders geeignet, da sie keine inflammatorischen Reaktionen oder Immunabstoßungen hervorrufen und ein geringes Risiko eines Gewebetraumas mit sich bringen. Zudem sind sie biologisch abbaubar und verfügen über zahlreiche Carbonsäuregruppen, die Chelatkomplexe mit mehrwertigen Kationen bilden bzw. chemisch vernetzt werden können. Sie sind vorteilhafterweise dazu in der Lage, unter Wasser und bei physiologischen Temperaturen zu vernetzen und können leicht in Lösung gehandhabt werden. Die Vernetzung erfolgt zügig aber ohne dabei feine Gewebsstrukturen des Patienten oder die Endoskopieinstrumente zu verkleben. Die entstehenden Gele weisen eine ausreichende Stabilität und Flexibilität auf, um zusammen mit den Partikeln extrahiert werden zu können.

Als Vernetzungsmittel der Komponente (B) dienen vorzugsweise geeignete Kationen. Vorteilhafterweise handelt es sich dabei in der Regel um natürlich in physiologischen Systemen vorkommende Kationen. Es müssen vorteilhafterweise keine zusätzlichen (aggressiven) Reagenzien zugegeben werden, um die Vernetzung unter physiologischen Bedingungen in Gang zu setzen. Zudem entstehen vorteilhafterweise keine unerwünschten Nebenprodukte.

Erfindungsgemäß bevorzugt sind solche Zusammensetzungen, die unter physiologischen Bedingungen abbinden können. Um stabile Vernetzungen über Kationenbrücken auszubilden, ist es vorteilhaft, wenn die Polymere der Komponente (A) über funktionelle Gruppen (in ausreichender Anzahl) verfügen, die auch bei (leicht) saurem pH als negativ geladene Einheiten vorliegen. In manchen Zusammensetzungen können beispielsweise der Vernetzungsgrad oder die Geschwindigkeit der Vernetzung über beeinflussbare Größen wie Konzentrationen oder pH-Werte der einzelnen Komponente gesteuert werden.

Zusätzlich oder alternativ ist bzw. sind das bzw. ein, mehrere oder sämtliche Vernetzungsmittel der Komponente (B) vorzugsweise ausgewählt aus der Gruppe bestehend aus zwei- und dreiwertigen Kationen, vorzugsweise aus Blei-, Kupfer-, Barium-, Strontium-, Cadmium-, Calcium-, Cobalt-, Nickel-, Zink-, Mangan- und Magnesium-Ionen, besonders bevorzugt aus Eisen- und Calcium-Ionen.

Eisen- und Calcium-Ionen sind in physiologischen Systemen natürlich vorkommende Kationen, die in Form von biologisch verträglichen Lösungen einfach verabreicht werden können. Sie haben eine geeignete Koordinationschemie und können stabile Chelatkomplexe zur Vernetzung ausbilden.

Erfindungsgemäß kann der Farbstoff ein Polymer umfassen, wobei sich das Polymer des Farbstoffes und das bzw. eines der vernetzbare(n) Polymer(e) der Komponente (A) entsprechen können. In diesem Fall beruhen das bzw. eines der vernetzbare(n) Polymer(e) der Komponente (A) und das Polymer des Farbstoff auf den gleichen Polymeren, wobei auf die Art der Monomere, aus denen diese Polymere aufgebaut sind, und vorzugsweise nicht auf die Anzahl der Monomere abzustellen ist.

Eine erfindungsgemäße Zusammensetzung kann zusätzlich weitere Komponenten enthalten. Beispielsweise können den Komponenten (A) und/oder (B) und/oder einer oder mehreren weiteren Komponenten einer erfindungsgemäßen Zusammensetzung Substanzen, die die Gelbildung und/oder die Einbindung der Partikel, fördern, zugegeben werden. Solche Substanzen können z.B. Quervernetzer zur Erhöhung der Stabilität des Gels sein.

Erfindungsgemäß ist es daher bevorzugt, dass die Komponente (A), die Komponente (B), und/oder (eine) weitere, gegebenenfalls in der Zusammensetzung enthaltene(n) Komponente(n) eine oder mehrere Substanzen zum Verbessern der Vernetzung des bzw. der vernetzbaren Polymere, insbesondere Quervernetzer, vorzugsweise Aminosäuren, enthält.

Vorzugsweise beträgt die Konzentration des/der in der Komponente (B) enthaltenen Vernetzungsmittel(s), bezogen auf das Gesamtvolumen der Komponente (B), wenigstens 0,01 mol/L und liegt vorzugsweise im Bereich von 0,01 bis 3,0 mol/L, besonders bevorzugt im Bereich von 0,1 bis 1,0 mol/L. Sollte eines der Vernetzungsmittel auch einen Farbstoff, wie hierin beschrieben, darstellen, so wird dieses Vernetzungsmittel zur Berechnung der Konzentration des/der in der Komponente (B) enthaltenen Vernetzungsmittel(s) als solches berücksichtigt.

Komponente (B) kann neben den genannten Vernetzungsmitteln auch weitere Bestandteile beinhalten. Zur Berechnung der Gesamtmenge des / der Vernetzungsmittel(s) in Komponente (B), dient die gesamte Komponente (B) samt der neben dem / den Vernetzungsmittel(n) ebenfalls vorhandenen Bestandteilen als Basis zur Berechnung.

Die Menge der vorhandenen Vernetzungsmittel beeinflusst sowohl die Geschwindigkeit der Vernetzungsreaktion, als auch die Stabilität und Flexibilität des vernetzten Gels. Die in der oben beschriebenen, bevorzugten Ausführung der vorliegenden Erfindung angegebenen Mengen gewährleisten, dass möglichst schnell ein stabiles und flexibles Gel gebildet wird, welches gegebenenfalls in einem Stück aus dem Körper entfernt werden kann.

Gemäß einer weiteren bevorzugten Ausgestaltung der vorliegenden Erfindung weist die Komponente (A) und/oder die Komponente (B) einen sauren pH-Wert auf, vorzugsweise einen pH-Wert im Bereich von 3,5 bis 4,5. Gemäß einer weiteren bevorzugten Ausgestaltung der vorliegenden Erfindung weist die Komponente (A) und/oder die Komponente (B) einen neutralen pH-Wert auf, vorzugsweise einen pH-Wert im Bereich von 6,5 bis 8,0, besonders bevorzugt einen pH-Wert im Bereich von 7,0 bis 7,5.

Bei einem pH im Bereich von 3,5 bis 4,5 liegen die Kationen der Komponente (B) frei in Lösung vor und stehen somit zur Komplexierung zur Verfügung. Vorteilhafterweise sind in diesem pH-Bereich aber die am Polysaccharid befindlichen Säuregruppen zu einem großen Teil deprotoniert, wodurch es zu einer effektiven Vernetzungsreaktion kommt. Wird im Bereich der zu entfernenden Partikel, eine gepufferte Lösung (bei einem pH von ca. 4) vorgelegt, kommt es infolge des Einleitens der vorzugsweise polysaccharidhaltigen Komponente (A) zu einer Herabsetzung der Löslichkeit. Dieser Prozess beansprucht eine gewisse Zeit, während der die zu entfernenden Partikel eingebettet werden. Vorteilhafterweise ist damit auch die Geschwindigkeit der Vernetzungsreaktion über den pH der verwendeten Komponenten steuerbar.

Ein neutraler pH-Wert hat sich insbesondere als unschädlich für das Körpergewebe erwiesen.

Erfindungsgemäß bevorzugt enthält die Zusammensetzung zudem magnetisierbare Teilchen, wobei

die magnetisierbaren Teilchen Bestandteil der Komponente (A) und/oder Bestandteil der Komponente (B) sind
und/oder wobei

die Zusammensetzung zudem Komponente (C) umfasst, die magnetisierbare Teilchen enthält,

so dass das vernetzte Gel zudem magnetisierbare Teilchen enthält.

Der Zusatz magnetisierbarer Teilchen eröffnet eine neue und vorteilhafte Methode, das gefestigte Gel durch Ausnutzung der magnetischen Eigenschaften aus dem Körper zu entfernen. Beispielsweise kann eine Magnetangel oder insbesondere ein magnetisches Fangkörbchen verwendet werden, welches die Vorteile eines Ankers und eines herkömmlichen Fangkörbchens verbindet.

Gemäß einer bevorzugten Ausführung der vorliegenden Erfindung sind die magnetisierbaren Teilchen ausgewählt aus Teilchen enthaltend oder bestehend aus ferromagnetische(n) Elemente(n) wie Eisen, Nickel und Kobalt sowie Legierungen wie AlNiCo, SmCo, $Nd_2Fe_{14}B$, $Ni_{80}Fe_{20}$, NiFeCo und/oder deren Oxide wie Eisenoxidpartikel, insbesondere Eisenoxidnanopartikel aus $Fe_3O_4$ und/oder $\gamma\text{-}Fe_2O_3$.

Gemäß einer weiteren bevorzugten Ausführung der vorliegenden Erfindung enthält das vernetzte Gel keine magnetisierbaren Teilchen.

Für den Fall, dass das vernetzte Gel zudem magnetisierbare Teilchen enthält, gilt vorzugsweise, dass die magnetisierbaren Teilchen die Farbe und/oder Färbung des Gels nicht bzw. nicht maßgeblich negativ im Sinne der Erfindung beeinflussen, bevorzugt nicht bzw. nicht maßgeblich beeinflussen.

Eisenoxidpartikel haben sich in medizintechnischen und pharmazeutischen Anwendungen, z.B. als intravenös verabreichtes Kontrastmittel für die Magnetresonanztomographie oder zur Tumortherapie, als geeignet erwiesen. Zur Erhöhung der Biokompatibilität und kolloidalen Stabilität sind solche Partikel meist umhüllt mit z.B. Dextranen, Polyvinylalkoholen, Dimercaptobernsteinsäure u.a..

Bevorzugt beträgt die Gesamtmenge der magnetisierbaren Teilchen, bezogen auf das Gesamtgewicht der die magnetisierbaren Teilchen enthaltenden Komponente (A) bzw. (B) bzw. (C), wenigstens 0,1 Gew.-% und liegt vorzugsweise im Bereich von 0,1 bis 70 Gew.-%, besonders bevorzugt im Bereich von 1 bis 11 Gew.-%. Um zu gewährleisten, dass das Gel umfassend die Partikel effizient durch Ausnutzung magnetischer Wechselwirkungen aus dem Körper entfernt werden kann, ist es notwendig, eine geeignete Menge an magnetisierbaren Teilchen im vernetzen Gel einzusetzen. Sollte eines der magnetisierbaren Teilchen auch einen Farbstoff, wie hierin beschrieben, darstellen, so wird dieses magnetisierbaren Teilchen zur Berechnung der Gesamtmenge der magnetisierbaren Teilchen als solches berücksichtigt. Vorzugsweise ist das / sind die magnetisierbare(n) Teilchen keine Farbstoffe wie hierin beschrieben.

Komponenten (A) und/oder (B) und/oder (C) (sofern vorhanden) können neben den genannten Bestandteilen auch weitere Bestandteile beinhalten. Zur Berechnung der Gesamtmenge der magnetisierbaren Teilchen in der diese Teilchen enthaltenen Komponente, wie oben beschrieben, dient die gesamte Komponente (A) bzw. (B) bzw. (C) samt der neben den genannten Bestandteilen ebenfalls vorhandenen Bestandteilen. Enthalten mehrere der Komponenten (A), (B) oder (C) magnetisierbare Teilchen, ist die Gesamtmenge der magnetisierbaren Teilchen in der jeweiligen Komponente bevorzugt für die jeweilige Komponente einzeln zu berechnen.

Sind in der erfindungsgemäßen Zusammensetzung neben der Komponente (A) und (B) noch mehrere weitere Komponenten vorhanden und umfasst eine der weiteren Komponenten magnetisierbare Teilchen wie oben beschrieben, so wird diese Komponente als Komponente (C) angesehen. Umfassen mehrere der weiteren Komponenten magnetisierbare Teilchen wie oben beschrieben und liegen diese mehreren Komponenten als getrennte Komponenten vor, so wird vorzugsweise die Komponente mit der höchsten Konzentration an magnetisierbaren Teilchen wie oben beschrieben als Komponente (C) angesehen.

Bevorzugt betrifft die Erfindung eine gelbildende Mehr-Komponenten-Zusammensetzung, vorzugsweise Zwei-Komponenten-Zusammensetzung, zur Bildung eines Gels,

die Zusammensetzung bestehend aus oder umfassend

eine Komponente (A), enthaltend ein oder mehrere vernetzbare, vorzugsweise kationisch vernetzbare Polymere, und

eine Komponente (B), enthaltend ein oder mehrere Vernetzungsmittel zum Vernetzen des bzw. der vernetzbaren Polymere

wobei Komponente (A) und Komponente (B) mindestens einen Farbstoff umfassen,

wobei der mindestens eine Farbstoff ein Farbstoff wie hierin beschrieben ist. Vorzugsweise ist der Farbstoff ausgewählt aus einer Kombination eines großen Moleküls, wie hierin beschrieben, und mindestens einer Markierung, wie hierin beschrieben oder einem kleinen Farbstoff, wie hierin beschrieben, zur Anwendung in einem Verfahren zum Entfernen von unerwünschten Partikeln aus einem Patienten.

Was oben zu den bevorzugten Ausführungsformen der Mehr-Komponenten-Zusammensetzung gesagt wurde, gilt auch hierfür entsprechend.

Ebenfalls hierin beschrieben ist ein vernetztes Gel bestehend aus oder umfassend ein oder mehrere Polymer(e), magnetisierbare Teilchen und mindestens einen Farbstoff, jeweils wie hierin beschrieben bzw. definiert,

wobei das vernetzte Gel herstellbar oder hergestellt ist durch

(i) Bereitstellen einer Komponente (A), enthaltend ein oder mehrere vernetzbare Polymere, vorzugsweise kationisch vernetzbare Polymere,

und Bereitstellen einer Komponente (B), enthaltend ein oder mehrere Vernetzungsmittel zum Vernetzen des bzw. der vernetzbaren Polymere,

sowie, optional, Bereitstellen einer Komponente (C) bzw. weiterer Komponente(n) (sofern vorhanden),

wobei die Komponente (A) und/oder die Komponente (B) und/oder, falls vorhanden, die Komponente (C) magnetisierbare Teilchen enthält,

(ii) in-Kontakt-Bringen der Komponenten (A) und (B) sowie gegebenenfalls (C) und weitere Komponenten (sofern vorhanden) unter Bedingungen, die eine Vernetzung des bzw. der vernetzbaren Polymere ermöglichen, so dass ein vernetztes Gel entsteht,

wobei Komponente (A) und/oder Komponente (B) mindestens einen Farbstoff umfassen,

wobei der mindestens eine Farbstoff eine transparente Färbung des Gels und optional der Komponente (A) und/oder der Komponente (B) vornimmt,

wobei der mindestens eine Farbstoff das Vernetzen des bzw. der vernetzbaren Polymere bei in-Kontakt-Bringen der Komponenten (A) und (B) nicht beeinträchtigt,

und wobei der mindestens eine Farbstoff aus dem gebildeten Gel nicht austritt.

Was hierin für die gelbildende Mehr-Komponenten-Zusammensetzung, insbesondere deren Vorteile und bevorzugte Ausgestaltungen, gesagt wurde, gilt entsprechend für das hierin beschriebene vernetzte Gel. Ebenfalls gilt das in Bezug auf Komponente (A) bzw. (B) bzw. (C) (sofern vorhanden) bzw. weitere Komponenten (sofern vorhanden) und deren jeweilige Bestandteile Gesagte entsprechend.

Komponente (A) und Komponente (B) umfassen mindestens einen Farbstoff und der/die Farbstoff(e) in Komponente (A) ist/sind von dem/den Farbstoff(en) in Komponente (B) unterschiedlich.

Vorzugsweise umfasst das Verfahren zur Herstellung des Gels, wie vorangehend beschrieben, zusätzlich den folgenden Schritt:
Anfärben der Komponente (A) und/oder (B) und/oder (C) (falls vorhanden) und/oder weitere Komponenten (sofern vorhanden) mit einem Farbstoff wie hierin beschrieben, insbesondere wie hierin als bevorzugt beschrieben.

Der Schritt des Anfärbens erfolgt bevorzugt vor dem in-Kontakt-bringen in Schritt (ii) des vorangehend beschriebenen Verfahrens.

Vorzugsweise ist bzw. sind das bzw. ein, mehrere oder sämtliche vernetzbare Polymere der Komponente (A) ausgewählt aus der Gruppe bestehend aus Polysacchariden, insbesondere Dextranen und Polysacchariden mit deprotonierten oder deprotonierbaren funktionellen Gruppen, vorzugsweise Carboxy-Gruppen, bevorzugt Dextranen und Polysacchariden aus der Gruppe der Polyuronide, besonders bevorzugt Dextranen und Polysacchariden aus der Gruppe der Alginate und Pektine.

Zusätzlich oder alternativ ist bzw. sind das bzw. ein, mehrere oder sämtliche Vernetzungsmittel der Komponente (B) vorzugsweise ausgewählt aus der Gruppe bestehend aus zwei- und dreiwertigen Kationen, vorzugsweise aus Eisen- und Calcium-Ionen.

Zusätzlich oder alternativ sind die magnetisierbaren Teilchen vorzugsweise ausgewählt aus Teilchen enthaltend oder bestehend aus ferromagnetische(n) Elemente(n) wie Eisen, Nickel und Kobalt sowie Legierun-

gen wie AlNiCo, SmCo, $Nd_2Fe_{14}B$, $Ni_{80}Fe_{20}$, NiFeCo und/oder deren Oxide wie Eisenoxidpartikel, insbesondere Eisenoxidnanopartikel aus $Fe_3O_4$ und/oder $\gamma$-$Fe_2O_3$.

Die magnetisierbaren Teilchen können auf beliebigem Weg in das gelbildende System gelangen. Sie können sowohl Bestandteil einer oder beider der Zusammensetzungen (A) und/oder (B) sein oder aber auch als Bestandteil einer weiteren Zusammensetzung (C) zugegeben werden.

Bevorzugt beträgt die Gesamtmenge der magnetisierbaren Teilchen, bezogen auf das Gesamtgewicht der die magnetisierbaren Teilchen enthaltenden Komponente (A) bzw. (B) bzw. (C), wenigstens 0,1 Gew.-% und liegt vorzugsweise im Bereich von 0,1 bis 70 Gew.-%, besonders bevorzugt im Bereich von 1 bis 11 Gew.-%. Sollte eines der magnetisierbaren Teilchen auch einen Farbstoff, wie hierin beschrieben, darstellen, so wird dieses magnetisierbaren Teilchen zur Berechnung der Gesamtmenge der magnetisierbaren Teilchen als solches berücksichtigt. Vorzugsweise ist das / sind die magnetisierbare(n) Teilchen keine Farbstoffe wie hierin beschrieben.

Komponenten (A) und/oder (B) und/oder (C) (sofern vorhanden) und/oder weitere Komponenten (sofern vorhanden) können neben den genannten Bestandteilen auch weitere Bestandteile beinhalten. Zur Berechnung der Gesamtmenge der magnetisierbaren Teilchen in der diese Teilchen enthaltenen Komponente, wie oben beschrieben, dient die gesamte Komponente (A) bzw. (B) bzw. (C) bzw. weitere Komponente(n) (sofern vorhanden) samt der neben den genannten Bestandteilen ebenfalls vorhandenen Bestandteilen. Enthalten mehrere der Komponenten (A), (B) oder (C) magnetisierbare Teilchen, ist die Gesamtmenge der magnetisierbaren Teilchen in der jeweiligen Komponente bevorzugt für die jeweilige Komponente einzeln zu berechnen.

Zusätzlich oder alternativ beträgt die Konzentration des/der in der Komponente (B) enthaltenen Vernetzungsmittel(s), bezogen auf das Gesamtvolumen der Komponente (B), vorzugsweise wenigstens 0,1 mol/L und liegt vorzugsweise im Bereich von 0,1 bis 3,0 mol/L, besonders bevorzugt im Bereich von 0,1 bis 1,0 mol/L. Sollte eines der Vernetzungsmittel auch einen Farbstoff, wie hierin beschrieben, darstellen, so wird dieses Vernetzungsmittel zur Berechnung der Konzentration des/der in der Komponente (B) enthaltenen Vernetzungsmittel(s) als solches berücksichtigt.

Komponente (B) kann neben den genannten Vernetzungsmitteln auch weitere Bestandteile beinhalten. Zur Berechnung der Gesamtmenge des / der Vernetzungsmittel(s) in Komponente (B), dient die gesamte Komponente (B) samt der neben dem / den Vernetzungsmittel(n) ebenfalls vorhandenen Bestandteilen.

Ebenfalls hierin beschrieben ist ein vernetztes Gel bestehend aus oder umfassend ein oder mehrere Polymer(e), magnetisierbare Teilchen und mindestens einen Farbstoff, jeweils wie hierin beschrieben bzw. definiert, wobei das vernetzte Gel herstellbar oder hergestellt ist durch

(i) Bereitstellen einer Komponente (A), enthaltend ein oder mehrere vernetzbare Polymere, vorzugsweise kationisch vernetzbare Polymere,

und Bereitstellen einer Komponente (B), enthaltend ein oder mehrere Vernetzungsmittel zum Vernetzen des bzw. der vernetzbaren Polymere,

sowie, optional, Bereitstellen einer Komponente (C) bzw. weiterer Komponente(n) (sofern vorhanden),

wobei die Komponente (A) und/oder die Komponente (B) und/oder, falls vorhanden, die Komponente (C) magnetisierbare Teilchen enthält,

(ii) in-Kontakt-Bringen der Komponenten (A) und (B) sowie gegebenenfalls (C) und weitere Komponenten (sofern vorhanden) unter Bedingungen, die eine Vernetzung des bzw. der vernetzbaren Polymere ermöglichen, so dass ein vernetztes Gel entsteht,

wobei Komponente (A) und Komponente (B) mindestens einen Farbstoff umfassen, wobei der mindestens einen Farbstoff ein Farbstoff wie hierin beschrieben ist. Vorzugsweise ist der Farbstoff ausgewählt aus einer Kombination eines großen Moleküls, wie hierin beschrieben, und mindestens einer Markierung, wie hierin beschrieben oder einem kleinen Farbstoff, wie hierin beschrieben.

Was oben zu den bevorzugten Ausführungsformen des vernetzten Gels bzw. der Mehr-Komponenten-Zusammensetzung gesagt wurde, gilt auch hierfür entsprechend.

Die vorliegende Erfindung betrifft eine Gelbildende Zusammensetzung wie hierin beschrieben zur Anwendung in einem Verfahren zum Entfernen von unerwünschten Partikeln aus einem Patienten.

Das vorstehend genannte Entfernen von unerwünschten Partikeln stell dabei ein invasives, chirurgisches Verfahren dar. Eine rein kosmetische Anwendung ist hiervon nicht betroffen. Das Entfernen von unerwünschten Partikeln wird gezielt durchgeführt, die unerwünschten Partikel werden dabei bewusst aus dem Körper entfernt.

Zu entfernende (unerwünschte) Partikel im Sinne der vorliegenden Erfindung können Partikel jeglicher Art sein, die aus medizinischer Sicht aus dem Körper des Patienten entfernt werden müssen oder können. Vorzugsweise beschreibt der Begriff "zu entfernende (unerwünschte) Partikel" alle zu entfernenden (unerwünschten) Partikel in einem Menschen oder Tier, ob selbst gebildet, durch externe Hilfe gebildet oder durch externen Eintrag immobilisiert. Solche Partikel können vorzugsweise gemeinsam entfernt werden.

Vorzugsweise sind die Partikel Ablagerungen, Ausfallprodukte, Fremdkörper und/oder Fragmente davon und/oder Splitter körpereigener Strukturen. Ablagerungen können insbesondere Harnsteine, Speichelsteine, Gallensteine, Bauchspeichelsteine oder Nierensteine sein. Unter Ausfallprodukten sind Partikel wie beispielsweise Gallensteine als Ausfallprodukt der Gallenflüssigkeit zu verstehen. Fremdkörper können beispielsweise mineralische Fragmente, Metall-, Kunststoff- oder Holzsplitter sein, die beispielsweise bei Verletzungen wie Schürfwunden in den Körper gelangen. Der Begriff "Fragmente davon" bezieht sich auf die vorgenannten Partikelformen und verdeutlicht, dass diese vor oder während der Entfernung in kleinere Teile bzw. Bruchstücke zerkleinert werden können. Auch ist hierbei möglich, dass durch die Anwendung nur Teile (Fragmente) entfernt werden, während die restlichen Fragmente in einem weiteren Schritt oder einem weiteren Verfahren entfernt werden. Splitter körpereigener Strukturen können beispielsweise bei Operationen, wie dem Einbringen oder Entfernen von Implantaten, entstehen und können daher beispielsweise Knochensplitter oder Fräsreste sein. Hierunter fallen bevorzugt aber auch Splitter anderer Strukturen wie z.B. Knorpelgewebe oder Tumorfragmente, die beispielsweise bei einem vorhergehenden oder gleichzeitig durchgeführten chirurgischen Eingriff entstanden sind. Der Begriff Splitter umfasst dabei Strukturen jeglicher Form und Größe.

Bevorzugt erfolgt das Entfernen der unerwünschten Partikel als ein Verfahren enthaltend die folgenden Schritte:

(i) Bereitstellen der Komponente (A) und (B) sowie gegebenenfalls (C) und weitere Komponente(n) (sofern vorhanden),

(ii) Einbringen der Komponente (A) und (B) sowie gegebenenfalls (C) und weitere Komponente(n) (sofern vorhanden) in den Körper des Patienten in einen Bereich der zu entfernende Partikel enthält, unter Bedingungen, die bei Kontakt der Komponente (A) mit der Komponente (B) eine Vernetzung des bzw. der vernetzbaren Polymere ermöglichen, so dass ein vernetztes Gel entsteht, das die zu entfernenden Partikel teilweise oder vollständig umschließt,

(iii) Entfernen des vernetzten Gels samt der davon umschlossenen Partikel aus dem Bereich des Körpers des Patienten.

Die Komponenten (A), (B) und gegebenenfalls (C) bzw. weitere Komponenten (sofern vorhanden) können in Schritt (ii) gleichzeitig oder nacheinander eingebracht werden. Auch können Komponenten vor Einbringen in den Körper gemischt werden. Einzelne Komponenten können auch vor Einbringen in den Körper gemischt werden, während andere Komponenten erst im Körper mit einem oder mehreren anderen Komponenten in Kontakt gebracht werden. Die Komponenten (A), (B) und gegebenenfalls (C) bzw. weitere Komponenten (sofern vorhanden) können auch gleichzeitig, aber separat, d.h. in voneinander getrennter Form, in den Körper eingebracht werden.

Vorzugsweise umfasst das Verfahren den folgenden weiteren Schritt, der zeitlich vor Schritt (ii) erfolgt: (i.2) Fragmentieren eines oder mehrerer Partikel in dem Bereich des Körpers des Patienten, so dass zwei oder mehrere, vorzugsweise eine Vielzahl von Fragmenten des/der Partikel(s) entsteht bzw. entstehen.

Wird dieser Schritt ausgeführt, können die Partikel bereits als Fragmente vorliegen, die durch diesen Schritt dann wiederum in Fragmente ihrerseits zerteilt werden.

Bevorzugt umfasst der Begriff "Vielzahl von Fragmenten" mindestens 5, vorzugsweise mindestens 10, besonders bevorzugt mindestens 15, ganz besonders bevorzugt mindestens 20 Fragmente. Liegt ein Partikel, der in diesem Schritt fragmentiert wird, bereits als Fragment vor, so bezieht sich der Begriff "Vielzahl von Fragmenten des/der Partikel(s)" auf den bereits fragmentierten Partikel und die Fragmente die aus diesem entstehen.

Das Verfahren kann zusätzlich oder alternativ den folgenden weiteren Schritt umfassen, der zeitlich vorzugsweise vor Schritt (ii) und vorzugsweise nach Schritt (i.2) (falls vorhanden) erfolgt: (i.3) Verbinden von zwei oder mehreren Partikeln bzw. zwei oder mehreren Fragmenten, jeweils wie oben beschrieben, oder von Partikeln und Fragmenten.

Vorzugsweise werden die verbundenen Partikel und/oder Fragmente weiterhin als Partikel und/oder Fragmente bezeichnet.

Figur 1 (links) zeigt eine Schweineniere, in welcher ein erfindungsgemäßes Gel mit Dextranblau (weißer Pfeil) und ein Vergleichsgel mit schwarzem Farbstoff (schwarzer Pfeil) hergestellt wurde. Figur 1 (rechts) zeigt die entfernten Gele, oben: transparent, erfindungsgemäß; unten: nicht transparent, nicht erfindungsgemäß.

Figur 2 zeigt die für die Versuche verwendeten Schweinenieren (links) bzw. eine aufgeschnittene Schweineniere (rechts).

Figur 3 zeigt eine Einwegspritze mit einer Komponente (A) gemäß der hierin beschriebenen Erfindung (links) und eine Einwegspritze mit einer nicht erfindungsgemäßen Komponente (A) (rechts); in diesem Zustand ist kein Unterschied zu sehen.

Figur 4 zeigt eine Schweineniere (links), in welcher ein erfindungsgemäßes Gel hergestellt wurde (Mitte, weißer Pfeil) und entfernt wurde (rechts). Keine Anfärbung des Nierengewebes ist zu erkennen.

Figur 5 zeigt eine Schweineniere (links) mit Nierensteinen, in welcher ein erfindungsgemäßes Gel hergestellt wurde, das die Nierensteine umschließt, (Mitte, weißer Pfeil) und entfernt wurde (rechts). Das entfernte Gel beinhaltet die Nierensteine.

Figur 6 zeigt eine Schweineniere, in welcher ein nicht erfindungsgemäßes Gel mit Brillantblau FCF hergestellt wurde (links oben, weißer Pfeil: noch nicht gefestigt; rechts oben, weißer Pfeil: gefestigt) und entfernt wurde (unten). Das Nierengewebe wurde angefärbt (unten; weiße Pfeile). Die Anfärbung hat sich auch durch Waschen (links unten) nicht entfernen lassen (rechts unten; weißer Pfeil).

Figur 7 zeigt eine Schweineniere (links) mit Nierensteinen, in welcher ein nicht erfindungsgemäßes Gel mit Brillantsäuregrün BS (auch Grün S genannt) hergestellt wurde (links: noch nicht gefestigt). Das Nierengewebe wurde angefärbt (rechts; weißer Pfeil, Klammer).

Figur 8 zeigt einen Vergleich der Versuche mit erfindungsgemäßen Zusammensetzungen (jeweils unten) und nicht erfindungsgemäßen Zusammensetzungen (jeweils oben). Bei den nicht erfindungsgemäßen Zusammensetzungen hat sich das Nierengewebe angefärbt (jeweils weißer Pfeil), links: Übersicht; rechts: Vergrößerung.

Im Folgenden wird die vorliegende Erfindung anhand ausgewählter Beispiele näher erläutert.

Beispiele

Beispiel 1: Herstellung von Komponenten (A), (B) und (C)

Zur Herstellung einer beispielhaften Komponente (A) werden 5 g Dextranblau und 4 g Natriumalginat in 1 L Wasser gelöst.

Zur Herstellung einer beispielhaften Komponente (B) werden 10 g Calciumchlorid-Dihydrat in 1 L Wasser gelöst.

Zur Herstellung einer beispielhaften Komponente (C) wird eine 4 bis 40 mM Eisen (0,35 bis 3,5 g pro Liter) enthaltende Partikel-Suspension in Wasser oder physiologischem Puffer zubereitet (M. Geppert et al., Nanotechnology 22 (2011) 145101). Diese Lösung wird zu 1% bis 50% zu A oder B gegeben.

Beispiel 2: Auswahl verschiedener Farbstoffe

2 g Alginat werden in 200 mL Wasser gelöst und mit verschiedenen Farbstoffen angefärbt. Komponente (B) wird gemäß Beispiel 1 hergestellt und mit Komponente (A) vermischt, so dass ein gefestigtes Gel entsteht.

Als Farbstoffe wurden verwendet: Farbe gelb (Riboflavin, E101ii, 29 mg/mL; Chinolingelb E104; Curcumin E100; Lutein E161 b; Tartrazin E102; Carotine E160; Sunset Yellow FCF E110), Farbe rot (echtes Karmin, E120, 29 mg/mL), Farbe blau (Brilliantblau, FCF E133, 2 mM). Farbe grün (Chlorophyllin, E141, 1 mM = 3 mg/mL). Farbe weiß (Titandioxid, E 171, 7 mg/mL). Farblos (kein Farbstoff).

Es zeigte sich, dass sich ein gefärbtes Gel mittels diverser Farbstoffe herstellen lässt. Besonders vorteilhaft waren die Gele, die in den Farben grün oder blau angefärbt wurden, da sich ein besonders guter Kontrast zu organischem Gewebe (Nierenschleimhaut) bildete und die Gele gut mit dem Auge unter Laborbeleuchtung erkannt werden konnten.

Als nächstes wurden zudem folgende Farbstoffe wie oben beschrieben getestet: Brillantblau FCF, Dextranblau, Methylenblau, Natrium-Kupfer-Chlorophyllin, Titandioxid, Trypanblau, Indigo Carmin. Von diesen Farbstoffen fällt nur Dextranblau unter die in Anspruch 1 beschriebene Liste aus angefärbten Molekülen.

Bei den Versuchen zeigt sich, dass die Biokompatibilität der Farben in den zu verwendenden Konzentrationen unterschiedlich ausfällt, dass die Farben unterschiedlich schnell aus einem Hydrogel herausdiffundieren können, dass die eingesetzten Farben einen negativen Einfluss auf die Gelbildung zeigen können und dass es ebenso zu einer Beeinflussung auf die Dynamik des Auflösens der Gele kommen kann.

Insbesondere Dextranblau und Natrium-Kupfer-Chlorophyllin erfüllten das Anforderungsprofil vollumfänglich.

Beispiel 3: Vergleich Brillantblau FCF, Patentblau V, Indiqokarmin, Anthocyane und Dextranblau

Eine Komponente (A) wurde entsprechend Beispiel 1 mit Dextranblau hergestellt. Weitere Komponenten (A) wurde entsprechend Beispiel 2 hergestellt, als Farbstoff wurden Brillantblau FCF (Dr. Oetker Back- und Speisefarbe "blau"), Patentblau V (E131), Indigokarmin E(132) bzw. Anthocyane E(163) verwendet.

Komponente (B) wurde entsprechend Beispiel 1 hergestellt und mit 0,023 g Riboflavin angefärbt.

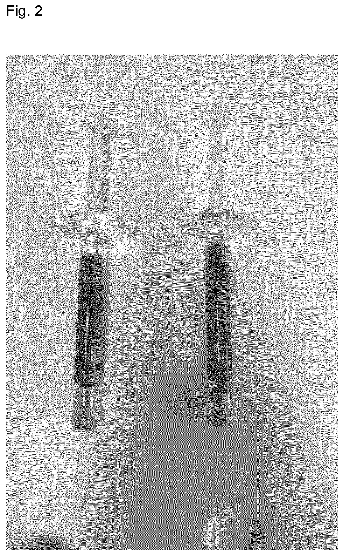

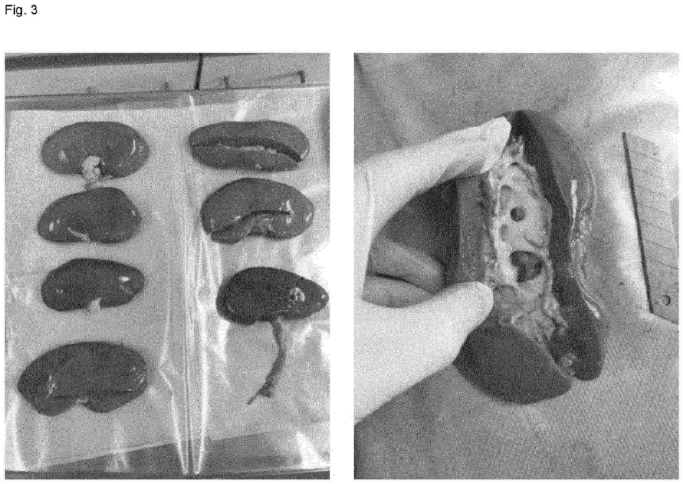

Beide Komponenten wurden mittels Einwegspritzen (Fig. 2) in Schweinenieren (Fig. 3) eingebracht, so dass sich ein gefestigtes Gel bildete. Die Gele wurden entfernt und die Schweinenieren wurden auf Anfärbung überprüft.

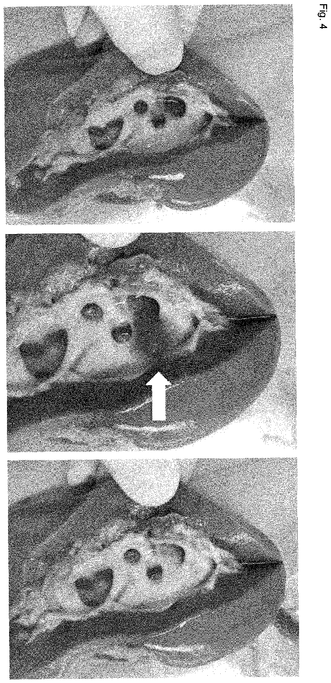

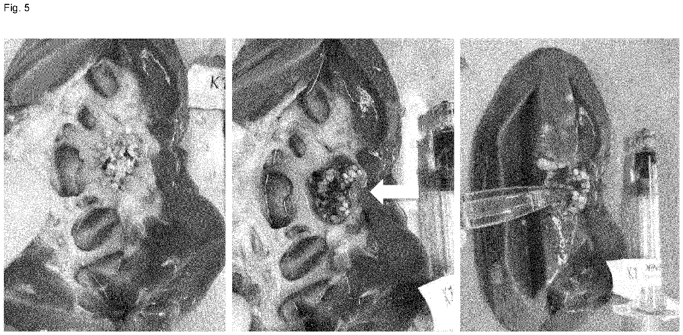

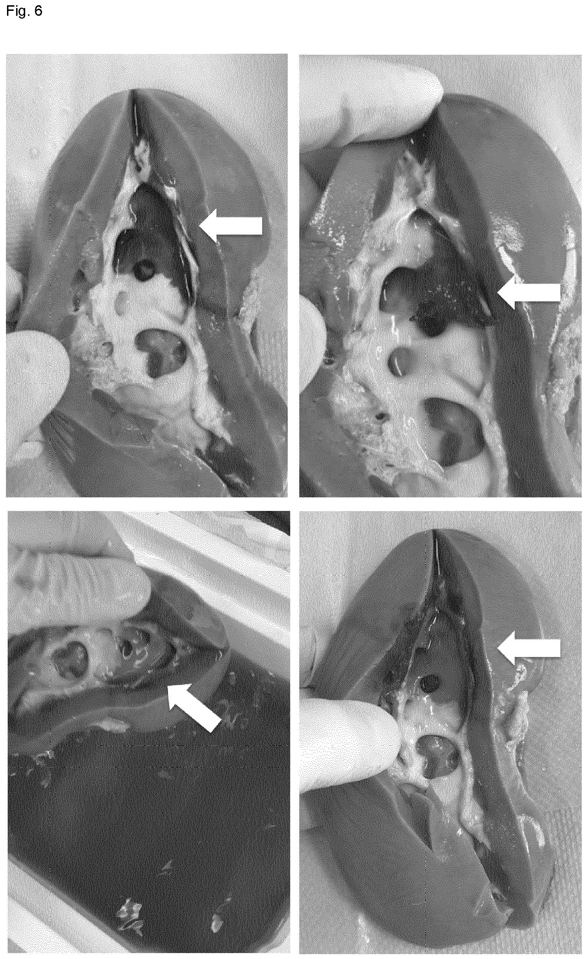

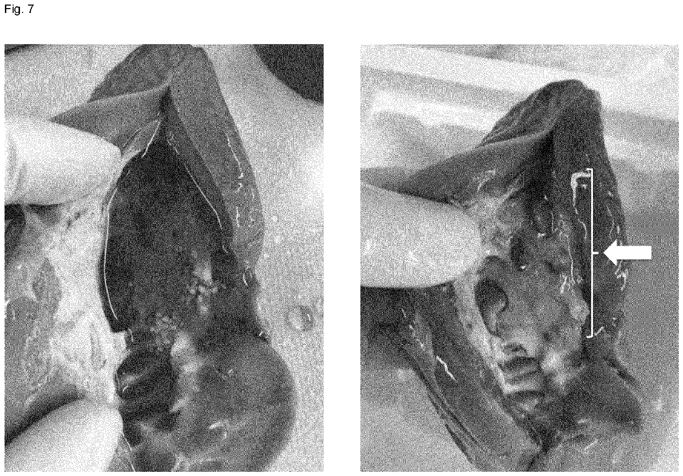

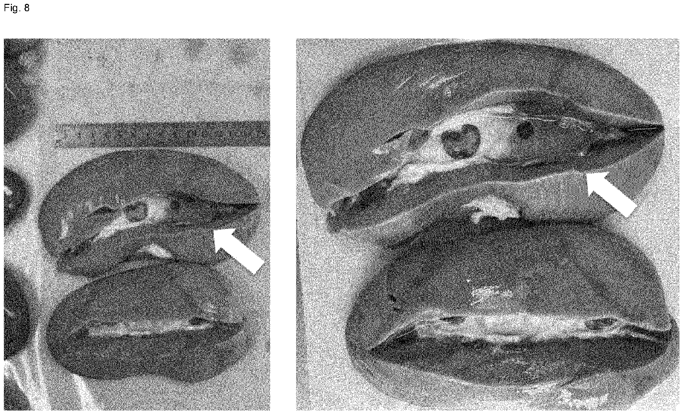

Es zeigte sich, dass Dextranblau alle Anforderungen erfüllte, ein transparent gefärbtes Gel ermöglicht und das umliegende Nierengewebe nicht anfärbt (Figs. 4 und 5), wohingegen die Farbstoffe Brillantblau FCF, Patentblau V, Indigokarmin und Anthocyane ungeeignet waren, da diese das umliegende Nierengewebe anfärbten (Figs. 6 und 7). Auch nach wiederholtem Abwaschen ließ sich die Farbe nicht von dem Nierengewebe entfernen (Fig. 6). Im Vergleich zeigt sich, dass Dextranblau geeignet war, Brillantblau FCF allerdings nicht (Fig. 8).

Von diesen Farbstoffen fällt nur Dextranblau unter die in Anspruch 1 beschriebene Liste aus angefärbten Molekülen.

Beispiel 4: Anwendung eines erfindungsgemäßen gelbildenden Systems zur Entfernung von Harnsteinen

Ein Zugang zum Harntraktlumen (z.B. zum Nierenbeckenkelchsystem) wird entweder ureterorenoskopisch (durch Harnröhre, Blase und Harnleiter) oder perkutan (durch Hautpunktion an der Flanke) geschaffen. Eine spezielle Schleuse (ggf. ein Polymerrohr mit Metallanteilen) mit einem Innendurchmesser von 3 bis 9 mm wird darin platziert. Über den vorgelegten Zugangsschaft wird ein Endoskop ins Harntraktlumen (z.B. ins Nierenbeckenkelchsystem) eingebracht, das Operationsgebiet inspiziert und der Harnstein bzw. die Harnsteine visualisiert. Der Harnstein bzw. die Harnsteine werden z. B. mittels eines Holmium-Lasers zertrümmert. Die großen und mittelgroßen Fragmente werden mit einem Steinfanginstrument entfernt. Anschließend wird ein Katheter über das Endoskopgerät (durch den Zugang) eingeführt und bis zu 3 mL, insbesondere 300 bis 500 µL einer Komponente (A) gemäß Beispiel 1 in den Bereich des Harntrakts (z.B. ins Nierenbeckenkelchsystem) so appliziert, dass A die Steine bzw. die Fragmente des bzw. der zertrümmerten Harnsteine(s) umschließt bzw. einbettet. Danach werden, ebenfalls über den im Endoskop befindlichen Katheter, bis zu 9 mL einer Komponente (B) gemäß Beispiel 1 in die Nähe von B appliziert. Eine aktive Vermischung von A mit B ist nicht notwendig. Innerhalb weniger Sekunden bis einer Minute kommt es zu einer Gelbildung. Der Katheter kann mit 0,9 % NaCl-Lösung gespült werden zwischen der Applikation von A und B. Dann wird ein Greifinstrument über das Operationsendoskop über die Zugangsschleuse eingeführt. Mit dem Greifinstrument wird das gefestigte Gel in einem Stück oder in mehreren Teilen gefasst und durch Extraktion aus dem Körper entfernt.

Beispiel 5: Messung der Transmission T zur Bestimmung von Transparenz

Eine Komponente (A) wurde entsprechend Beispiel 1 hergestellt. Komponente (B) wurde entsprechend Beispiel 1 hergestellt und mit 0,023 g Riboflavin angefärbt. Komponenten (A) und (B) wurden miteinander gemischt, woraufhin sich ein Gel gebildet hat.

Zur Messung der Transmission T wurde das Spektrum im sichtbaren Spektrum von 400-800 nm aufgenommen mit einer Schrittweite von 1 nm.

Die zu messende Substanz wird in Standardküvetten gegeben, so dass eine Schichtdicke von 10 mm erreicht wird:

Das Gel wird passend zurechtgeschnitten, in die Küvette eingebracht und ggf. mit weiterer Komponente (B) aufgefüllt, um etwaige Luftbläschen zu entfernen. Auch hier wird eine Schichtdicke von 10 mm erzielt.

Als Referenz wurde Wasser vermessen, das in die Küvette gefüllt wurde, so dass eine Schichtdicke von 10 mm erreicht wird.

Die Intensität jedes Messpunktes des Gels wird mit der Referenz (Wasser) verglichen und als Prozentualer Anteil zur Referenz dargestellt (Wasser = 100%).

Die Transmission T bestimmt sich als arithmetisches Mittel aller Teilintensitäten zwischen 400 und 800 nm in Prozent.

Zur Bestimmung der für Transparenz notwendigen Mindestwerte der Transmission T wurde ein Text mit schwarzen Buchstaben hinter der jeweiligen Küvette positioniert. Gele, bei denen die Buchstaben durch die befüllte Küvette noch erkennbar (= transparentes Gel) bzw. nicht mehr erkennbar (= intransparentes Gel) waren, wurden auf ihre Transmission T gemessen.

Bei dem erfindungsgemäßen Gel aus Komponenten (A) und (B), wie oben beschrieben, waren die schwarzen Buchstaben durch die gefüllte Küvette erkennbar (transparentes Gel). Für die Transmission T des Gels wurden in mehreren Versuchen Werte zwischen 19 und 25 % gemessen.

Weitere, nicht erfindungsgemäße Gele wurden hergestellt und wie oben beschrieben in Küvetten eingebracht. Jedoch waren die schwarzen Buchstaben durch die gefüllte Küvette nicht erkennbar (intransparente Gele). Eine Transmission T zwischen 12 % und 15 % wurde für diese Gele gemessen. Daraus folgt, dass Gele mit einer Transmission T von bis zu 15 % noch intransparent sind.

**Patentansprüche**

**1.** Gelbildende Mehr-Komponenten-Zusammensetzung, vorzugsweise Zwei-Komponenten-Zusammensetzung, zur Bildung eines Gels,

die Zusammensetzung bestehend aus oder umfassend

eine Komponente (A), enthaltend ein oder mehrere vernetzbare, vorzugsweise kationisch vernetzbare Polymere, und eine Komponente (B), enthaltend ein oder mehrere Vernetzungsmittel zum Vernetzen des bzw. der vernetzbaren Polymere,

wobei Komponente (A) und Komponente (B) mindestens einen Farbstoff umfassen, wobei der/die Farbstoff(e) in Komponente (A) unterschiedlich ist/sind von dem/den Farbstoff(en) in Komponente (B), wobei der mindestens eine Farbstoff eine transparente Färbung des Gels und optional der Komponente (A) und/oder der Komponente (B) vornimmt, wobei der mindestens eine Farbstoff das Vernetzen des bzw. der vernetzbaren Polymere bei in-Kontakt-Bringen der Komponenten (A) und (B) nicht beeinträchtigt,

wobei der mindestens eine Farbstoff aus dem gebildeten Gel nicht austritt,

und wobei der mindestens eine Farbstoff ein angefärbtes Molekül ausgewählt aus der Gruppe bestehend aus Dextranen, Polyethylenglycol, Stärke, Gellane, Po-Iyelektrolyte wie Poly-Ilysin und andere Polyaminosäuren, wie z. B. Poly(ornithin), Poly(arginin), Poly(histidin); Polypeptide und Proteine wie z.B. Gelatine, sowie Polysaccharide wie Chitosan, Alginate wie Natriumalginat, Polyglykolsäure (PGA), Polymilchsäure (PLA), Poly-2-hydroxy-butyrat (PHB), Polycaprolacton (PCL), Poly(milch-co-glykol) säure (PLGA), Protaminsulfat, Spermidin, Albumin, Carrageenan, Furcellaran, Pektin, Xanthan, Hyaluronsäure, Natriumcarboxymethylcellulose, Heparin, Heparansulfat, Cellulosederivate oder Natriumcarboxymethylcellulose, Chondroitinsulfat, Dermatansulfat und Dextransulfat beinhaltet oder daraus besteht, zur Anwendung in einem Verfahren zum Entfernen von unerwünschten Partikeln aus einem Patienten.

2. Gelbildende Zusammensetzung zur Anwendung nach Anspruch 1,

wobei das bzw. ein, mehrere oder sämtliche vernetzbare Polymere der Komponente (A) ausgewählt ist bzw. sind aus der Gruppe bestehend aus Polysacchariden, insbesondere Dextranen und Polysacchariden mit deprotonierten oder deprotonierbaren funktionellen Gruppen, vorzugsweise Carboxy-Gruppen, bevorzugt Dextranen und Polysacchariden aus der Gruppe der Polyuronide, besonders bevorzugt Dextranen und Polysacchariden aus der Gruppe der Alginate und Pektine,
und/oder
wobei das bzw. ein, mehrere oder sämtliche Vernetzungsmittel der Komponente (B) ausgewählt ist bzw. sind aus der Gruppe bestehend aus zwei- und dreiwertigen Kationen, vorzugsweise aus Eisen- und Calcium-Ionen.

3. Gelbildende Zusammensetzung zur Anwendung nach einem der vorangehenden Ansprüche, wobei die Konzentration des/der in der Komponente (B) enthaltenen Vernetzungsmittel(s), bezogen auf das Gesamtvolumen der Komponente (B), wenigstens 0,1 mol/L beträgt und vorzugsweise im Bereich von 0,1 bis 3,0 mol/L, besonders bevorzugt im Bereich von 0,1 bis 1,0 mol/L liegt.

4. Gelbildende Zusammensetzung zur Anwendung nach einem der vorangehenden Ansprüche, wobei die Komponente (B) einen neutralen pH-Wert auf, vorzugsweise einen pH-Wert im Bereich von 6,5 bis 8,0 besonders bevorzugt einen pH-Wert im Bereich von 7,0 bis 7,5.

5. Gelbildende Zusammensetzung zur Anwendung nach einem der vorangehenden Ansprüche, wobei die Zusammensetzung zudem magnetisierbare Teilchen enthält, wobei

die magnetisierbaren Teilchen Bestandteil der Komponente (A) und/oder Bestandteil der Komponente (B) sind
und/oder wobei
die Zusammensetzung zudem Komponente (C) umfasst, die magnetisierbare Teilchen enthält,

vorzugsweise wobei die magnetisierbaren Teilchen ausgewählt sind aus Teilchen enthaltend oder bestehend aus ferromagnetische(n) Elemente(n) wie Eisen, Nickel und Kobalt sowie Legierungen wie AlNiCo, SmCo, $Nd_2Fe_{14}B$, $Ni_{80}Fe_{20}$, NiFeCo und/oder deren Oxide wie Eisenoxidpartikel, insbesondere Eisenoxidnanopartikel aus $Fe_3O_4$ und/oder y-$Fe_2O_3$.

6. Gelbildende Zusammensetzung zur Anwendung nach Anspruch 5, wobei die Gesamtmenge der magnetisierbaren Teilchen, bezogen auf das Gesamtgewicht der die magnetisierbaren Teilchen enthaltenden Komponente (A) bzw. (B) bzw. (C), wenigstens 0,1 Gew.-% beträgt und vorzugsweise im Bereich von 0,1 bis 70 Gew.-%, besonders bevorzugt im Bereich von 1 bis 11 Gew.-% liegt.

7. Gelbildende Zusammensetzung zur Anwendung nach einem der vorangehenden Ansprüche, wobei die Komponente (A), die Komponente (B), gegebenenfalls die Komponente (C) und/oder eine weitere, gegebenenfalls in der Zusammensetzung enthaltenen Komponente (D) eine oder mehrere Substanzen zum Verbessern der Vernetzung des bzw. dervernetzbaren Polymere, insbesondere Quervernetzer, vorzugsweise Aminosäuren, enthält.

8. Gelbildende Zusammensetzung zur Anwendung nach einem der Ansprüche 1 bis 7,
wobei die Partikel Ablagerungen, Ausfallprodukte, Fremdkörper und/oder Fragmente davon und/oder Splitter körpereigener Strukturen sind.

9. Gelbildende Zusammensetzung zur Anwendung nach Anspruch 8, wobei das Verfahren die folgenden Schritte enthält:

(i) Bereitstellen der Komponente (A) und (B) sowie gegebenenfalls (C),
(ii) Einbringen der Komponente (A) und (B) sowie gegebenenfalls (C) in den Körper des Patienten in einen Bereich der zu entfernende Partikel enthält,

unter Bedingungen, die bei Kontakt der Komponente (A) mit der Komponente (B) eine Vernetzung des bzw. der vernetzbaren Polymere ermöglichen, so dass ein vernetztes Gel entsteht, das die zu entfernenden Partikel teilweise oder vollständig umschließt,

(iii) Entfernen des vernetzten Gels samt der davon umschlossenen Partikel aus dem Bereich des Körpers des Patienten.

10. Gelbildende Zusammensetzung zur Anwendung nach einem der Ansprüche 8 oder 9,
    wobei das Verfahren den folgenden weiteren Schritt umfasst, der zeitlich vor Schritt (ii) erfolgt:
    Fragmentieren eines oder mehrerer Partikel in dem Bereich des Körpers des Patienten, so dass zwei oder mehrere, vorzugsweise eine Vielzahl von Fragmenten des/der Partikel(s) entsteht bzw. entstehen.

## Claims

1. Gel-forming multi-component composition, preferably two-component composition, for forming a gel,

   the composition comprising or consisting of

   a component (A), comprising one or more cross-linkable, preferably cationically cross-linkable polymer(s), and
   a component (B), comprising one or more cross-linking agent(s) for cross-linking the cross-linkable polymers,

   wherein component (A) and component (B) comprise at least one dye, wherein the dye(s) in component (A) is/are different from the dye(s) in component (B),
   wherein the at least one dye provides a transparent staining of the gel and optionally of component (A) and/or component (B),
   wherein the at least one dye does not impair the cross-linking of the cross-linkable polymer(s) when contacting components (A) and (B),
   wherein the at least one dye does not leak from the formed gel,
   and wherein the at least one dye comprises or consists of a stained molecule selected from the group consisting of dextrans, polyehtlyene glycol, starch, gellans, polyelectrolytes such as poly-l-lysine and other poly-amino acids such as poly(orni-thine), poly(arginine), poly(histidine); polypeptides and proteins such as gelatin, as well as polysaccharides such as chitosan, alginates such as sodium alginate, poly(glycolic acid) (PGA), poly(lactic acid) (PLA), poly-2-hydroxy butyrate (PHB), polycaprolactone (PCL), poly(lactic-co-glycolic) acid (PLGA), protamine sulfate, spermidine, albumin, carrageenan, furcellarane, pectin, xanthan, hyaluronic acid, sodium carboxymethyl cellulose, heparin, heparane sulfate, cellulose derivatives or sodium carboxymethyl cellulose, chondroitine sulfate, dermatan sulfate and dextran sulfate,
for use in a method for removing undesired particles from a patient.

2. Gel-forming composition for use according to claim 1,

   wherein the, one, more or all cross-linkable polymer(s) of component (A) is/are selected from the group consisting of polysaccharides, particularly dextrans and polysaccharides with deprotonized or deprotonizable functional groups, preferably carboxy groups, preferably dextrans and polysaccharides from the group of polyuronides, particularly preferably dextrans and polysaccharides from the group of alginates and pectins,
   and/or
   wherein the, one, more or all cross-linking agent(s) of component (B) is/are selected from the group consisting of bi- or tri-valent cations, preferably iron and calcium ions.

3. Gel-forming composition for use according to any of the preceding claims, wherein the concentration of the cross-linking agent(s) present in component (B), related to the total volume of component (B), is at least 0.1 mol/L and is preferably in the range of from 0.1 to 3.0 mol/L, particularly preferably in the range of from 0.1 to 1.0 mol/L.

4. Gel-forming composition for use according to any of the preceding claims, wherein component (B) has a neutral pH value, preferably a pH value in the range of from 6.5 to 8.0, particularly preferably a pH in the range of from 7.0 to 7.5.

5. Gel-forming composition for use according to any of the preceding claims, wherein the composition further comprises magnetisable particles, wherein

   the magnetisable particles are a component of component (A) and/or component of component (B),
   and/or wherein
   the composition further comprises component (C), which contains magnetisable particles,

   preferably wherein the magnetisable particles are selected from particles comprising or consisting of ferromagnetic element(s) such as iron, nickel, and cobalt, as well as alloys such as AlNiCo, SmCo, $Nd_2Fe_{14}B$, $Ni_{80}Fe_{20}$, NiFeCo, and/or their oxides such as iron oxide particles, particularly iron oxide particles from $Fe_3O_4$ and/or $\gamma$-$Fe_2O_3$.

6. Gel-forming composition for use according to claim 5, wherein the total amount of the magnetisable particles, related to the total weight of the components (A), (B), or respectively, (C) containing the magnetisable particles, is at least 0.1 wt.-% and is preferably in the range of from 0.1 to 70 wt.-%, particularly preferably in the range of from 1 to 11 wt.-%.

7. Gel-forming composition for use according to any of the preceding claims, wherein component (A), component (B), optionally component (C), and/or a further component (D) optionally present in the composition comprises one or more substances for improving the cross-linking of the cross-linkable polymer(s), particularly cross-linkers, preferably amino acids.

8. Gel-forming composition for use according to any of claims 1 to 7, wherein the particles are deposits, precipitation products, foreign particles and/or fragments thereof and/or splinters of endogenous structures.

9. Gel-forming composition for use according to claim 8, wherein the method comprises the following step(s):

   (i) providing component (A) and (B) as well as optionally (C),
   (ii) introducing components (A) and (B) as well as optionally (c) into the body of the patient in a area comprising particles to be removed, under conditions allowing a cross-linking of the cross-linkable polymer(s) under contact of component (A) with component (B), such that a cross-linked gel is formed, which partially or fully encloses the particle(s) to be removed,
   (iii) removing the cross-linked gel including the particles enclosed therein from the area of the body of the patient.

10. Gel-forming composition for use according to any of claims 8 or 9,
    wherein the method further comprises the step, which is performed temporally before step (ii):
    fragmenting one or more particle(s) in the area of the body of the patient, such that two or more, preferably a plurality of fragments of the particle(s) is/are obtained.

## Revendications

1. Composition multicomposante gélifiante, de préférence composition bicomposante, destinée à former un gel,

   la composition consistant en ou comprenant

      un composant (A) contenant un ou plusieurs polymères réticulables, de préférence réticulables par voie cationique, et un composant (B) contenant un ou plusieurs agents de réticulation pour réticuler le ou bien les polymère(s) réticulable(s),

   dans laquelle le composant (A) et le composant (B) comprennent au moins un colorant, dans laquelle le/les colorant(s) dans le composant (A) est/sont différent(s) du/des colorant(s) dans le composant (B),
   dans laquelle ledit au moins un colorant provoque une coloration transparente du gel et en option du composant (A) et/ou du composant (B),
   dans laquelle ledit au moins un colorant n'influence pas défavorablement la réticulation du ou bien des polymères réticulables lorsque les composants (A) et (B) sont mis en contact,
   dans lequel ledit au moins un colorant ne s'échappe pas du gel formé,
   et dans lequel ledit au moins un colorant contient une molécule colorée choisie dans le groupe constitué par les dextranes, le polyéthylène glycol, l'amidon, les gommes gellanes, les polyélectrolytes tels que la poly-l-lysine et d'autres polyaminoacides, tels que par exemple le poly(ornithine), le poly(arginine), le poly(histidine); les polypeptides et les protéines telles que la gélatine, ainsi que les polysaccharides tels que le chitosane, les alginates tels que l'alginate de sodium, l'acide polyglycolique (PGA), l'acide polylactique (PLA), le poly-2-hydroxybutyrate (PHB), la polycaprolactone (PCL), l'acide poly(lactique-co-glycolique) (PLGA), le sulfate de protamine, la spermidine, l'albumine, le carraghénane, la furcellarane, la pectine, la xanthane, l'acide hyaluronique, la carboxyméthylcellulose sodique, l'héparine, le sulfate d'héparane, les dérivés de cellulose ou la carboxyméthylcellulose sodique, le sulfate de chondroïtine, le sulfate de dermatane et le sulfate de dextrane, ou se compose de ceux-ci,
   destinée à être utilisée dans un procédé destiné à éliminer des particules indésirables d'un patient.

2. Composition gélifiante destinée à être utilisée selon la revendication 1,

dans lequel le ou bien un, plusieurs ou tous les polymères réticulables du composant (A) est ou bien sont choisi(s) dans le groupe constitué par les polysaccharides, en particulier les dextranes et les polysaccharides avec des groupes fonctionnels dé-protonés ou déprotonables, de préférence des groupes carboxy, de préférence des dextranes et des polysaccharides du groupe des polyuronides, de manière particulièrement préférée des dextranes et des polysaccharides du groupe des alginates et des pectines, et/ou

dans lequel le ou bien un, plusieurs ou tous les agents de réticulation du composant (B) est ou bien sont choisi(s) dans le groupe constitué de cations divalents et trivalents, de préférence d'ions de fer et de calcium.

3.  Composition gélifiante destinée à être utilisée selon l'une quelconque des revendications précédentes, dans laquelle la concentration du/des agent(s) de réticulation contenu(s) dans le composant (B), par rapport au volume total du composant (B) est d'au moins 0,1 mol/L et de préférence se situe dans la plage allant de 0,1 à 3,0 mol/L, de manière particulièrement préférée dans la plage allant de 0,1 à 1,0 mol/L.

4.  Composition gélifiante destinée à être utilisée selon l'une quelconque des revendications précédentes, dans laquelle le composant (B) présente une valeur de pH neutre, de préférence une valeur de pH comprise entre 6,5 et 8,0, de manière particulièrement préférée une valeur de pH comprise entre 7,0 et 7,5.

5.  Composition gélifiante destinée à être utilisée selon l'une quelconque des revendications précédentes, dans laquelle la composition contient en outre des particules magnétisables, dans laquelle

    les particules magnétisables font partie du composant (A) et/ou font partie du composant (B)
    et/ou dans laquelle
    la composition comprend en outre un composant (C) qui contient des particules magnétisables,

    de préférence dans laquelle les particules magnétisables sont choisies parmi des particules contenant ou constituées d'élément(s) ferromagnétique(s) tel(s) que le fer, le nickel et le cobalt ainsi que des alliages tels que AlNiCo, SmCo, $Nd_2Fe_{14}B$, $Ni_{80}Fe_{20}$, NiFeCo et/ou leurs oxydes tels que les particules d'oxyde de fer, en particulier des nanoparticules d'oxyde de fer constituées de $Fe_3O_4$ et/ou de $\gamma$-$Fe_2O_3$.

6.  Composition gélifiante destinée à être utilisée selon la revendication 5, dans laquelle la quantité totale des particules magnétisables, par rapport au poids total du composant (A) ou bien (B) ou bien (C) contenant les particules magnétisables, est d'au moins 0,1 % en poids et de préférence se situe dans la plage allant de 0,1 à 70 % en poids, de manière particulièrement préférée dans la plage allant de 1 à 11 % en poids.

7.  Composition gélifiante destinée à être utilisée selon l'une quelconque des revendications précédentes, dans laquelle le composant (A), le composant (B), le cas échéant le composant (C) et/ou un autre composant (D) contenu le cas échéant dans la composition contient/contiennent une ou plusieurs substances pour améliorer la réticulation du ou bien des polymères réticulables, en particulier des agents de cross-linking, de préférence des acides aminés.

8.  Composition gélifiante destinée à être utilisée selon l'une quelconque des revendications 1 à 7, dans laquelle les particules sont des dépôts, des produits de précipitation, des corps étrangers et/ou des fragments de ceux-ci et/ou des éclats de propres structures du corps.

9.  Composition gélifiante destinée à être utilisée selon la revendication 8, dans laquelle le procédé comprend les étapes suivantes consistant à:

    (i) fournir les composants (A) et (B) et, le cas échéant, (C),
    (ii) introduire les composants (A) et (B) et, le cas échéant, (C) dans le corps du patient dans une zone qui contient des particules à éliminer, dans des conditions qui, lorsque le composant (A) entre en contact avec le composant (B), permettent une réticulation du ou bien des polymère(s) réticulable(s), de sorte qu'il se forme un gel réticulé qui entoure en partie ou complètement les particules à éliminer,
    (iii) éliminer le gel réticulé, y compris les particules qu'il entoure, de la zone du corps du patient.

10. Composition gélifiante destinée à être utilisée selon l'une quelconque des revendications 8 ou 9, dans laquelle le procédé comprend l'étape supplémentaire suivante qui a lieu avant l'étape (ii): fragmenter une ou plusieurs particule(s) dans la zone du corps du patient de sorte que deux ou plusieurs, de préférence une pluralité de fragments de la ou des particule(s) soit ou bien soient créé(s).

Fig. 1

Fig. 2

Fig. 3

Fig. 4

Fig. 5

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5244913 A
- WO 2005037062 A
- US 20080065012 A
- US 6663594 B2
- WO 2014173467 A
- WO 2014173468 A

**In der Beschreibung aufgeführte Nicht-Patentliteratur**

- **CLOUTIER J** ; **CORDEIRO ER** ; **KAMPHUIS GM** ; **VILLA L** ; **LETENDRE J** ; **DE LA ROSETTE JJ** ; **TRAXER O**. The glueclot technique: a new technique description for small calyceal stone fragments removal. *Urolithiasis*, 2014, vol. 42 (5), 441-444
- **M. GEPPERT et al.** *Nanotechnology*, 2011, vol. 22, 145101